(19) Europäisches Patentamt
European Patent Office
Office européen des brevets

(11) **EP 4 401 035 A1**

(12) **EUROPEAN PATENT APPLICATION**

(43) Date of publication:
**17.07.2024 Bulletin 2024/29**

(21) Application number: **23305037.6**

(22) Date of filing: **11.01.2023**

(51) International Patent Classification (IPC):
**G06T 7/11** *(2017.01)*    **G06T 7/136** *(2017.01)*

(52) Cooperative Patent Classification (CPC):
**G06T 7/11; G01R 33/5608; G06T 7/136;**
G01R 33/56341; G06T 2207/10088;
G06T 2207/20128; G06T 2207/30016

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Dassault Systèmes**
**78140 Vélizy-Villacoublay (FR)**

(72) Inventor: **MOREAU, Louise**
**Vélizy-Villacoublay (FR)**

(74) Representative: **Bandpay & Greuter**
**11 rue Christophe Colomb**
**75008 Paris (FR)**

(54) **SEGMENTING A HUMAN PATIENT TRACTOGRAM**

(57) The disclosure notably relates to a computer-implemented method for segmenting of a human patient tractogram into one or more white matter streamline bundles, comprising obtaining a tractogram of a human patient, the tractogram including tractogram streamlines, and a white matter atlas including one or more bundles each including respective atlas streamlines. The method also comprises, for at least one bundle of the atlas and its respective atlas streamlines, attributing, to the at least one bundle, respective tractogram streamlines, the respective tractogram streamlines including one or more first sets each of at least one tractogram streamline, each first set corresponds to a respective set of at least one atlas streamline of the at least one bundle, and the respective tractogram streamlines further including one or more second sets each of at least one tractogram streamline, each second set corresponds to a respective sectional portion of a respective set of at least one atlas streamline of the at least one bundle.

EP 4 401 035 A1

# Description

## TECHNICAL FIELD

The disclosure relates to the field of computer programs and systems, and more specifically to a method, program and system for segmenting a human patient tractogram into one or more white matter streamline bundles.

## BACKGROUND

The modelling of the white matter of the brain (consisting of fibers that conduct electrical pulses) may be used for assisting clinicians to perform diagnostics around a common representation of contextualized white-matter data in order to take a decision about the treatment or monitoring of a pathology. A human patient tractogram comprises tractogram streamlines which represent partially the white matter. However, there are some issues for obtaining the tractogram streamlines. An issue is that a tractogram streamline may be erroneously cut, e.g., a part of the tractogram streamline may be erroneously considered as entering grey matter, leading to the prematurely cutting the tractogram streamline. Another issue arises in that there may be noise present in the acquisition of the streamlines. For example, the streamline may have a wrong direction and stop suddenly. Another issue arises in that the segmentation suffers from dimensionality problems. The tractogram may comprise millions of streamlines, resulting in that analyzing the anatomical accuracy of all of the streamlines is computationally expensive.

Within this context, there is still a need for an improved method for segmenting a human patient tractogram into one or more white matter streamline bundles.

## SUMMARY

It is therefore provided a computer-implemented method for segmenting a human patient tractogram into one or more white matter streamline bundles. The method comprises obtaining a tractogram of a human patient The tractogram includes tractogram streamlines. The method also comprises obtaining a white matter atlas including one or more bundles each including respective atlas streamlines. The method also comprises, for at least one bundle of the atlas and its respective atlas streamlines, attributing, to the at least one bundle, respective tractogram streamlines, the respective tractogram streamlines including one or more first sets each of at least one tractogram streamline. Each first set corresponds to a respective set of at least one atlas streamline of the at least one bundle. The respective tractogram streamlines further include one or more second sets each of at least one tractogram streamline, where each second set corresponds to a respective sectional portion of a respective set of at least one atlas streamline of the at least one bundle.

The method may comprise one or more of the following:

- the attributing comprises:

  ∘ using a predetermined clustering algorithm to obtain a plurality of tractogram streamline clusters;
  ∘ using the predetermined clustering algorithm to obtain a plurality of atlas streamline clusters;
  ∘ selecting the one or more first sets from the plurality of tractogram streamline clusters, a respective tractogram streamline cluster being selected as a first set when the respective tractogram streamline cluster fulfills a proximity criterion with the plurality of atlas streamline clusters; and
  ∘ selecting the one or more second sets from the plurality of tractogram streamline clusters, a respective tractogram streamline cluster being selected as a second set when the respective tractogram streamline cluster fulfills the proximity criterion with a respective plurality of sectional portions of the atlas streamline clusters;

- selecting the one or more first sets comprises:

  ∘ computing a tractogram centroid for each respective tractogram streamline cluster using a predetermined centroid computation algorithm;
  ∘ computing a first atlas centroid for each respective atlas streamline cluster using the predetermined centroid computation algorithm; and
  ∘ identifying each tractogram streamline cluster for which the value of a predetermined distance between its tractogram centroid and each first atlas centroid is not above a predetermined threshold; and
  selecting the one or more second sets comprises, for each remaining tractogram streamline cluster:
  ∘ determining a plurality of second atlas centroids each for a respective sectional portion of the respective plurality of sectional portions of the atlas streamline clusters; and
  ∘ identifying each tractogram streamline cluster for which the value of the predetermined distance between its tractogram centroid and each second atlas centroid is not above a predetermined threshold;

- determining the plurality of second atlas centroids comprises:

  ∘ determining the value of a length of the tractogram centroid;
  ∘ for each first atlas centroid having a value of the length higher than the tractogram centroid, extracting one or more sectional portions of the first atlas centroid having a same value of the length as the tractogram centroid;

- extracting the one or more sectional portions of the first atlas centroid comprises cutting iteratively the first atlas centroid using a predetermined offset;
- the predetermined clustering algorithm comprises for a respective plurality of streamlines:

  ○ assigning an initial streamline to an initial streamline cluster;
  ○ iteratively visiting subsequent streamlines of the respective plurality of streamlines; and
  ○ for each respective subsequent streamline and with respect to a predetermined distance:

    ▪ computing a respective distance value between the subsequent streamline and a centroid of each already-existing streamline cluster; and
    ▪ determining a respective streamline cluster having a smallest distance value:

      • if the respective distance value is below a predetermined threshold, assigning the respective subsequent streamline to the respective streamline cluster;
      • else, creating a subsequent streamline cluster and assigning the respective subsequent streamline to said subsequent streamline cluster.

- the predetermined clustering algorithm further comprises, after assigning all streamlines of the plurality of streamlines:

  ○ re-computing the centroid of each streamline cluster using the predetermined centroid computation algorithm; and
  ○ for each streamline assigned to a respective streamline cluster, and with respect to the predetermined distance:

    ▪ computing a respective distance value between the streamline and the centroid of the respective streamline cluster;
    ▪ un-assigning the streamline from the respective streamline cluster if the respective distance value is above the predetermined threshold;

  ○ re-computing again the centroid of each streamline cluster using the predetermined centroid computation algorithm; and
  ○ for each streamline un-assigned to another respective streamline cluster, and with respect to the predetermined distance:

    ▪ computing a respective distance value between the streamline and the centroid of each streamline cluster;
    ▪ determining a streamline cluster having a smallest distance value:

      • if the respective distance value is below the predetermined threshold, re-assigning the unassigned streamline to the determined streamline cluster;
      • else creating a subsequent streamline cluster and re-assigning the subsequent streamline to a subsequent streamline cluster;

- the predetermined distance is a minimum direct-flip distance;
- the predetermined clustering algorithm takes as input a threshold value, and prior to the steps of using the predetermined clustering algorithm and to the steps of selecting the one or more first sets and the one or more second sets, the method comprises:

  ○ retrieving all the tractogram streamlines from the tractogram and all atlas streamlines from the white matter atlas;
  ○ applying the predetermined clustering algorithm to said all the tractogram streamlines to obtain an initial plurality of tractogram streamline clusters;
  ○ applying the predetermined clustering algorithm to said all the atlas streamlines; and
  ○ selecting one or more initial sets of tractogram streamlines from the plurality of initial tractogram streamline clusters, a respective initial tractogram streamline cluster being selected as an initial set when the respective initial tractogram streamline cluster fulfills a coarser proximity criterion with the plurality of initial atlas streamline clusters;

the using of the predetermined clustering algorithm to obtain the plurality of tractogram streamline clusters being applied to the streamlines of at least part of the one or more initial sets of tractogram streamlines;

- further comprises, to obtain the at least part of the one or more initial sets:

  ○ computing a binary mask from the white matter atlas, the binary mask comprising binary-valued voxels, wherein a binary-valued voxel has a value of one when said voxel intersects a part of one atlas streamlines from the retrieved all white matter atlas streamlines and a value of zero otherwise; and

∘ applying the binary mask to exclude each initial set of tractogram streamlines having a centroid comprising at least one point in a voxel having a value of zero;

- obtaining the tractogram comprises:

    ∘ obtaining a diffusion magnetic resonance image (MRI) of the brain;
    ∘ determining a diffusion voxel model from the diffusion MRI; and
    ∘ constructing the tractogram from the diffusion voxel model;

- the result of the attributing is usable for diagnosing and/or treating a patient with respect to a medical condition, wherein optionally the at least one bundle comprises:

    ∘ a bundle corresponding to the cingulum fasciculus, and/or the medical condition is a condition impacting functioning of memory, such as the Alzheimer disease,
    ∘ a bundle corresponding to the corpus callosum, and/or the medical condition is the Hungtington disease,
    ∘ a bundle corresponding to optical radiations, and/or the medical condition is a condition impacting the optical nerve fibers and/or incurring an optic neuritis, such as multiple sclerosis,
    ∘ a bundle corresponding to the corticospinal tracts and/or the medical condition is the Parkinson disease, and/or
    ∘ one or more bundles usable for neurosurgical planning.

It is further provided a computer program comprising instructions for performing the method.
It is further provided a computer readable storage medium having recorded thereon the computer program.
It is further provided a system comprising a processor coupled to a memory, the memory having recorded thereon the computer program.

## BRIEF DESCRIPTION OF THE DRAWINGS

Non-limiting examples will now be described in reference to the accompanying drawings, where:

- FIG. 1 shows a flowchart of the method;
- FIG. 2 shows an example of the system;
- FIG. 3 shows a flowchart of an example of part of the method;
- FIG. 4 shows an example of a part of the method;
- FIG. 5 shows another flowchart of part of the method; and
- FIG.s 6 to 18 show examples of the method.

## DETAILED DESCRIPTION

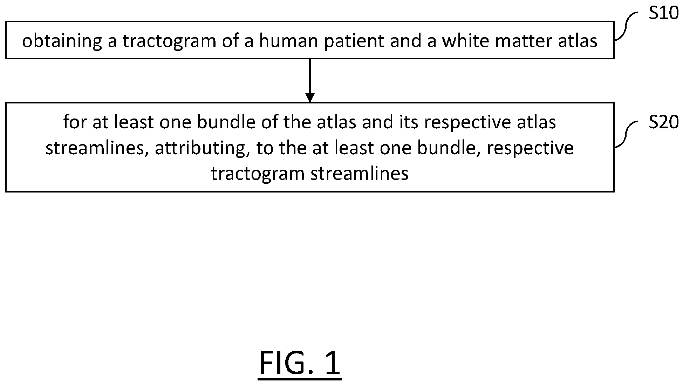

With reference to the flowchart of FIG. 1, it is proposed a computer-implemented method for segmenting a human patient tractogram into one or more white matter streamline bundles. The method comprises obtaining a tractogram of a human patient. The tractogram includes tractogram streamlines. The method also obtains a white matter atlas. The white matter atlas includes one or more bundles, each bundle including respective atlas streamlines. The method also comprises for at least one bundle of the atlas and its respective atlas streamlines, attributing, to the at least one bundle, respective tractogram streamlines. The respective tractogram streamlines (of the at least one bundle) include one or more first sets, each set being a set of at least one tractogram streamline. Each first set corresponds to a respective set of at least one atlas streamline of the at least one bundle. The respective tractogram streamlines (of the at least one bundle) further include one or more second sets each of at least one tractogram streamline. Each second set corresponds to a respective sectional portion of a respective set of at least one atlas streamline of the at least one bundle.
Such a method improves the segmentation of a human patient tractogram into one or more white matter streamline bundles.
Notably, the method attributes, to the at least one bundle of the atlas, respective tractogram streamlines which anatomically correspond to the atlas streamlines of the at least one bundle included in the white matter atlas. Each bundle of the atlas may represent fibers belonging to a same anatomical segment of the white matter for the atlas, such that the method allows to identify in the human patient tractogram streamlines representing fibers belonging to the same anatomical segment for the human patient (real person to whom the tractogram processed by the method belongs). Such identification can in turn be applied to different medical uses. The method may perform such attributing for several bundles of the atlas, for example each bundle present in the atlas, such that the method allows to detect different anatomical parts of the white matter of the subject human patient.
The method performs such identification of the respective tractogram streamlines for the at least atlas one bundle with an improved accuracy. On the one hand, each first set corresponds to a respective set of at least one atlas streamline of the at least one bundle, and thus each first set represents an anatomically corresponding set of one or more entire fibers of white matter. On the other hand, each second set corresponds to a respective sectional portion of a respective set of at least one atlas streamline of the at least one bundle, and thus each second set represents an anatomically corresponding sectional portion of a set of one or more fibers of white matter, e.g., up to a certain length or proportion with respect to the actual anatomical dimensions of the fibers. Thus, the method ensures that, for example, sectional portions of tractogram streamlines, which are still anatomically accurate although possibly incomplete, are still represented in the segmentation or in the second sets. In other words, the method provides robustness to the fact that in the human patient tractogram, some streamlines are cut erroneously with respect to corresponding white matter fibers, whereas the corresponding atlas streamlines are not cut in such a way. By looking at sectional portions of atlas streamline sets, the method reduces false negatives with respect to attributing tractogram streamlines to the at least one bundle.

In addition, the method performs the attributing by looking at correspondences between sets of streamlines and/or sectional portions thereof. In examples, at least one (e.g., several or each) first set may comprise several tractogram streamlines, and at least one (e.g., several or each) second set may comprise several tractogram streamlines, and, similarly, at least one (e.g., several or each) first-set-corresponding set of at least one atlas streamline of the at least one bundle may comprise several atlas streamlines, and at least one (e.g., several or each) second-set-corresponding set of at least one atlas streamline of the at least one bundle may comprise several atlas streamlines. Such a set-to-set correspondence approach makes the method robust to tractogram-acquisition noise, and also greatly reduces computational complexity.

The method is computer-implemented. This means that steps (or substantially all the steps) of the method are executed by at least one computer, or any system alike. Thus, steps of the method are performed by the computer, possibly fully automatically, or, semi-automatically. In examples, the triggering of at least some of the steps of the method may be performed through user-computer interaction. The level of user-computer interaction required may depend on the level of automatism foreseen and put in balance with the need to implement user's wishes. In examples, this level may be user-defined and/or pre-defined.

A typical example of computer-implementation of a method is to perform the method with a system adapted for this purpose. The system may comprise a processor coupled to a memory and a graphical user interface (GUI), the memory having recorded thereon a computer program comprising instructions for performing the method. The memory may also store a database. The memory is any hardware adapted for such storage, possibly comprising several physical distinct parts (e.g. one for the program, and possibly one for the database).

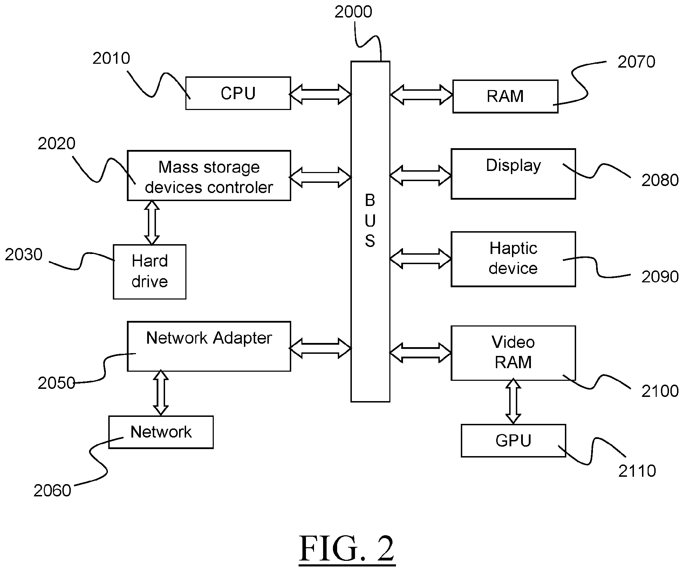

FIG. 2 shows an example of the system, wherein the system is a client computer system, *e.g.* a workstation of a user.

The client computer of the example comprises a central processing unit (CPU) 2010 connected to an internal communication BUS 2000, a random access memory (RAM) 2070 also connected to the BUS. The client computer is further provided with a graphical processing unit (GPU) 2110 which is associated with a video random access memory 2100 connected to the BUS. Video RAM 2100 is also known in the art as frame buffer. A mass storage device controller 2020 manages accesses to a mass memory device, such as hard drive 2030. Mass memory devices suitable for tangibly embodying computer program instructions and data include all forms of nonvolatile memory, including by way of example semiconductor memory devices, such as EPROM, EEPROM, and flash memory devices; magnetic disks such as internal hard disks and removable disks; magneto-optical disks. Any of the foregoing may be supplemented by, or incorporated in, specially designed ASICs (application-specific integrated circuits). A network adapter 2050 manages accesses to a network 2060. The client computer may also include a haptic device 2090 such as cursor control device, a keyboard or the like. A cursor control device is used in the client computer to permit the user to selectively position a cursor at any desired location on display 1080. In addition, the cursor control device allows the user to select various commands, and input control signals. The cursor control device includes a number of signal generation devices for input control signals to system. Typically, a cursor control device may be a mouse, the button of the mouse being used to generate the signals. Alternatively or additionally, the client computer system may comprise a sensitive pad, and/or a sensitive screen.

The computer program may comprise instructions executable by a computer, the instructions comprising means for causing the above system to perform the method. The program may be recordable on any data storage medium, including the memory of the system. The program may for example be implemented in digital electronic circuitry, or in computer hardware, firmware, software, or in combinations of them. The program may be implemented as an apparatus, for example a product tangibly embodied in a machine-readable storage device for execution by a programmable processor. Method steps may be performed by a programmable processor executing a program of instructions to perform functions of the method by operating on input data and generating output. The processor may thus be programmable and coupled to receive data and instructions from, and to transmit data and instructions to, a data storage system, at least one input device, and at least one output device. The application program may be implemented in a high-level procedural or object-oriented programming language, or in assembly or machine language if desired. In any case, the language may be a compiled or interpreted language. The program may be a full installation program or an update program. Application of the program on the system results in any case in instructions for performing the method. The computer program may alternatively be stored and executed on a server of a cloud computing environment, the server being in communication across a network with one or more clients. In such a case a processing unit executes the instructions comprised by the program, thereby causing the method to be performed on the cloud computing environment.

The method is for segmenting a human patient tractogram into one or more white matter streamline bundles. This means that the method takes the human patient tractogram as input and outputs an enriched version thereof where tractogram streamlines are attributed to one or more (different) segment(s). Such a segmentation may in turn allow the method to further generate and optionally display one or more 3D representations of white matter fiber bundles represented by the tractogram streamlines. The displaying may include, for example, displaying only the 3D representations of white matter (thereby excluding other 3D representations of tractogram streamlines of the tractogram not output by the method) and/or highlighting (e.g., with a different color or with a label) the 3D representations of white matter fiber bundles represented by the tractogram streamlines output by the method.

The human patient tractogram may be a 3D structure including tractogram streamlines. A tractogram streamline may be a numeric piece of data that represents a solid 3D line, for example as a sequence of 3D points. The sequence of 3D points may comprise for example 100 or more, even 400 or more 3D points. In other words, the streamline may represent a discretization of the solid 3D line, as sampled by the sequence of 3D points. The tractogram streamlines may be constructed from a diffusion voxel model determined from a diffusion Magnetic Resonance Image (MRI).

Obtaining S10 the tractogram may comprise acquiring (i.e., physically measuring) and/or obtaining a diffusion MRI of the brain. The diffusion MRI may be obtained from physical measurements of the diffusion of water molecules within the brain performed via a diffusion magnetic resonance imaging process. White matter is a structure within the brain which is composed of fibers that gather into bundles. Thus, the water molecules present in the white matter spread within a constrained environment. Hence, the measurement of the diffusion of water molecule within the brain corresponds to finding the structure of the white matter.

The obtaining S10 may also comprise determining the diffusion voxel model from the diffusion MRI. The diffusion voxel model may be a set of voxels, wherein each voxel comprises indication of the diffusion of water molecules within the brain. The diffusion voxel model may be modeled from a mathematical model that determines the diffusion of water molecules from the diffusion MRI on each voxel. The diffusion voxel model may be obtained from a Fiber Orientation Density Function (FOD), which provides a probability of the water molecule to spread into one preferential direction. Hence, a tractogram streamline represents the 3D line resulting from the computed probability that the water spreads into one preferential direction.

The obtaining S10 may also comprise constructing the tractogram from the diffusion voxel model.

In other words, the obtaining S10 may gather the tractogram streamlines resulting from the diffusion voxel model which represent the computed probability that the water spreads into one preferential direction within the brain, as captured by the diffusion MRI. The construction of the tractogram may be performed by choosing an initial position in the diffusion MRI (also called seed position). The initial position may be chosen in an area of the diffusion MRI representing white-matter. The construction may determine a main diffusion direction at the initial position using the diffusion voxel model. The tractogram streamlines may be constructed, e.g., as sequences of 3D points traversing the voxels of the diffusion voxel model, to follow this main diffusion direction until it reaches a stopping criteria or other voxel.

A white matter atlas may be a 3D structure including one or more bundles (e.g., set or collection of) each including respective atlas streamlines. Each respective atlas streamlines represent the fibers comprised in bundles of white matter. In other words, the atlas streamlines have anatomical meaning in that these are representative of the fibers composing white matter. The white matter atlas may be obtained at S10 from a predetermined collection of white matter bundles constructed from multiple patients, e.g., as constructed from the Tractometer Challenge (which may be consulted on the website https://tractometer.org/ismrm2015/home/).

The obtaining S10 of the tractogram and the white matter atlas may for example comprise downloading/retrieving (e.g. through a network) said tractogram from a (e.g., distant) memory or server or any non-volatile storage where the tractogram may have been stored. Alternatively, the tractogram may be obtained independently (e.g., at different times, from different locations and/or by different entities) from the white matter atlas, e.g., the white matter atlas may be downloaded/retrieved another memory or server or any non-volatile storage than the one where the tractogram may have been stored.

For at least one bundle of the atlas and its respective atlas streamlines, the method attributes S20, to the at least one bundle, respective tractogram streamlines. By attributing, it is meant that the at least one bundle is associated (linked) to respective tractogram streamlines. In other words, the attributing at S20 marks the respective tractogram streamlines as corresponding to said at least one bundle, e.g., with a label or other data indicative of the association. Such marking or labeling data may be created and written on memory. The method may comprise after the attributing S20, storing on persistent (non-volatile) memory the initial tractogram enriched with the result of the attributing S20 (e.g., said marking or labeling data).

The respective tractogram streamlines (attributed to the at least one bundle) include one or more first sets each of at least one tractogram streamline, i.e., the union of the one or more first sets forming a collection of at least one tractogram streamline, for example 10, 100 or even 1000 or more tractogram streamlines.

Each first set of at least one tractogram streamline corresponds to a respective set of at least one atlas streamline of the at least one bundle. In other words, the collection of the at least one tractogram streamline is linked to the respective set of at least one atlas streamline of the at least one bundle, for example through a label or any other type of data indicative of the association.

The respective tractogram streamlines further include one or more second sets. Each of the one or more second sets include at least one tractogram streamline, for example 10, 100 or even 1.000 or more tractogram streamlines. Each second set corresponds to a respective sectional portion of a respective set of at least one atlas streamline of the at least one bundle. By "sectional portion", it is meant a portion of the respective set between two cross-sections of the respective set, e.g. between a beginning "cross-section" and an end "cross-section", between the beginning "cross-section" and an intermediate cross-section, or between two intermediate cross-sections. The cross-sections may be set by convention, e.g., at point within the 3D line of a corresponding at least one atlas streamline and/or a boundary (such as an endpoint) of the at least one atlas streamline in the white matter atlas. As fibers of the white matter are elongated, so are atlas streamlines, and the at least one bundle comprises atlas streamlines that can be grouped into elongated sets each of at least one streamline. The second sets of tractogram streamline(s) correspond to sectional portions of such elongate sets of atlas streamline(s). In other words, such an elongate set of atlas streamline(s) is cut/sectioned at two different positions along its length, and the length-portion thereby obtained is put into correspondence with tractogram streamlines to form a second set.

This improves the segmentation of a human patient tractogram into one or more white matter streamline bundles. Indeed, as the method for obtaining tractogram streamlines from a diffusion voxel model is inherently probabilistic (and also may be affected by a noisy acquisition of the diffusion MRI), there may be tractogram streamlines that might not have an anatomical meaning, in other words, not representative of the fibers composing white matter, or which appear due to a noisy acquisition of the tractogram. Thanks to attributing, to the at least one bundle of the white matter atlas (which are endowed with anatomical meaning) respective tractogram streamlines, the method maintains tractogram streamlines that have a correct anatomical meaning, thereby improving accuracy of the segmentation resulting from the maintained tractogram streamlines.

In addition, each second set corresponds to a respective sectional portion of a respective set of at least one atlas streamline of the at least one bundle. Hence, the method also maintains cross-sectional portions of tractogram streamlines which still have anatomical meaning.

Optionally, the method may display the one or more first sets and the one or more second sets as the one or more 3D representations of white matter fiber bundles represented by the tractogram streamlines. For example, the one or more first sets one and more second sets may be highlighted from other tractogram streamlines to the user. Alternatively, the method may exclude any tractogram streamline not selected as the first sets one or more second sets in the displaying.

The result of the attributing may be usable for diagnosing and/or treating a patient with respect to a medical condition. In other words, the method may comprise using the result of the segmentation to perform a diagnosis step and/or a treatment step of which at least one parameter depends on said segmentation. For example, the method may comprise displaying to one or more medical practitioners a 3D representation of the segmentation, for example showing/highlighting the tractogram streamlines attributed to (e.g., each) at least one bundle (including the one or more first sets and the one or more second sets) in a (e.g., respective) different color. Alternatively or additionally, the output of S20 may be fed to an automatic process to perform such use.

Optionally, the at least one bundle may comprise a bundle corresponding to the cingulum fasciculus. Additionally or alternatively, the medical condition is a condition impacting functioning of memory, such as the Alzheimer disease. For example, the method may be used for extracting fractional anisotropy of bundles of the cingulum to determine cognitive dysfunction and atrophy of the limbic system on Alzheimer's disease patients.

Additionally or alternatively, the at least one bundle may comprise a bundle corresponding to the corpus callosum. Additionally or alternatively the medical condition is the Hungtington disease. For example, the method may be used for examining corpus callosum pathways, which may be compromised prior to disease onset.

Additionally or alternatively, the at least one bundle may comprise a bundle corresponding to optical radiations. Additionally or alternatively the medical condition is a condition impacting the optical nerve fibers and/or incurring an optic neuritis, such as multiple sclerosis, for example the method may be used for determining abnormalities within optic radiations.

Additionally or alternatively, the at least one bundle may comprise a bundle corresponding to the corticospinal tracts. Additionally or alternatively the medical condition is the Parkinson disease. The method may be used for example for determining disease-specific regional white matter changes in parkinsonian disorders.

Additionally or alternatively, the at least one bundle may comprise one or more bundles usable for neurosurgical planning. For example, the method may be used for correlating the segmentation with clinical information such as patient outcome, clinical functional testing, or electro-cortical stimulation, and performing neurosurgical planning based on the result of the correlation.

The attributing S20 may comprise using a predetermined clustering algorithm to obtain a plurality of tractogram streamline clusters. A tractogram streamline cluster may be a set (or collection) of tractogram streamlines. The predetermined clustering algorithm may be a set of instructions which assigns (or selects) tractogram streamlines from the tractogram to a respective tractogram streamline cluster. The predetermined clustering algorithm may select the tractogram streamlines based on a similarity criteria. For example, the predetermined clustering algorithm may assign tractogram streamlines to a tractogram streamline cluster by determining tractogram streamlines having a similar geometrical disposition (e.g., having a similar degree of curvature). The predetermined clustering algorithm may associate the determined tractogram streamlines in the tractogram streamline cluster to data pieces indicative of its association with the tractogram streamline cluster.

The attributing S20 may also comprise using the predetermined clustering algorithm to obtain a plurality of atlas streamline clusters. In other words, the attributing may use the same predetermined clustering algorithm used for obtaining the plurality of tractogram streamline clusters. An atlas streamline cluster may thus be a set (or collection) of atlas streamlines, which are determined based on the same criteria as the tractogram streamline clusters are obtained.

The attributing S20 may also comprise selecting the one or more first sets from the plurality of tractogram streamline clusters. In other words, a first set may be a cluster from the tractogram streamline clusters. A respective tractogram streamline cluster may be selected as a first set when the respective tractogram streamline cluster fulfills a proximity criterion with the plurality of atlas streamline clusters. By proximity criterion it is meant any criterion for determining a proximity (with respect to the shape, similarity or location) between the tractogram streamline clusters and a plurality of atlas streamline clusters. The proximity criterion may be based on a satisfaction of a predetermined threshold, that is, the attributing may determine that a tractogram streamline cluster satisfies the proximity criterion when the proximity is not above (equal or lower than) a predetermined threshold. The proximity may be determined by any function which may take into account a geometrical overlap between tractogram streamline clusters and respective atlas streamline clusters, for example a distance between streamlines of included in the tractogram streamline clusters and respective atlas streamline clusters. The method thus selects sets from the tractogram streamline clusters overlapping substantially with respect to respective atlas streamlines, e.g., an overlap above a predetermined threshold.

The attributing S20 may also comprise selecting the one or more second sets from the plurality of tractogram streamline clusters. A respective tractogram streamline cluster may be selected as a second set when the respective tractogram streamline cluster fulfills the proximity criterion with a respective plurality of sectional portions of the atlas streamline clusters.

A respective tractogram streamline cluster may be selected as a second set when the respective tractogram streamline cluster fulfills a proximity criterion with the plurality sectional portions of the atlas streamline clusters. The proximity criterion may further take into account the proximity (with respect to the shape, similarity or location) between sectional portions of the tractogram streamline clusters and a plurality of sectional portions of atlas streamline clusters. For example, the proximity criterion may take into account a geometrical overlap between sectional portions of tractogram streamline clusters and respective sectional portions, e.g., between beginning "cross-section" or end "cross-sections", of atlas streamline clusters.

This improves the accuracy of the segmentation. Indeed, thanks to selecting the one or more first sets and the one or more second sets when the respective tractogram streamlines fulfill the proximity criterion, the method ensures that the resulting segmentation (comprising the first and second sets) is representative of the anatomy of the white matter (as represented in the white matter atlas).

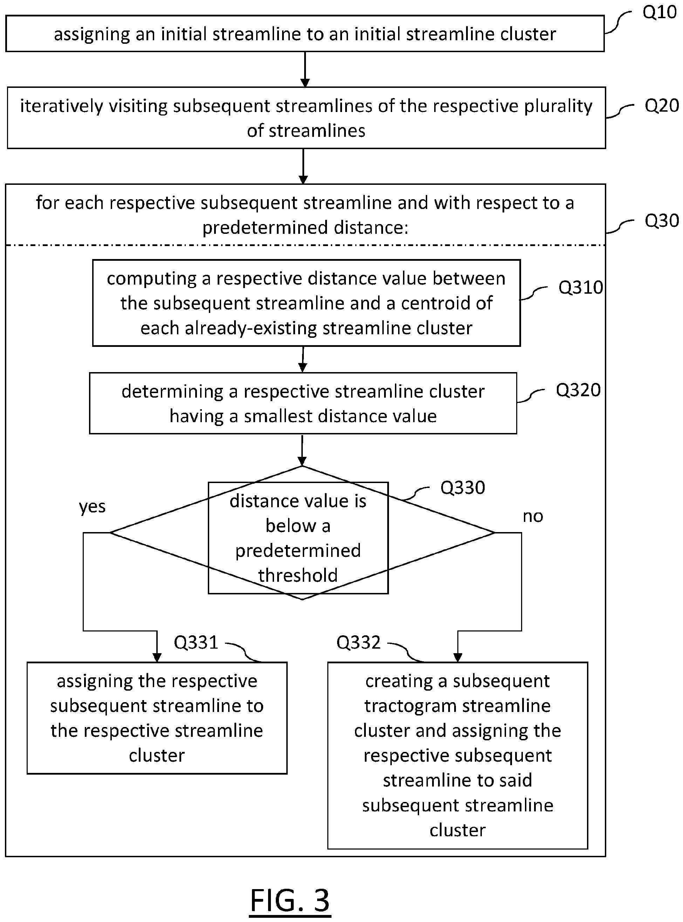

The predetermined clustering algorithm is now discussed, with reference to FIG. 3i. As mentioned previously, it is to be understood that the predetermined clustering algorithm may be applied to either (both of) tractogram streamlines and/or to atlas streamlines to obtain a respective plurality of tractogram streamline clusters and/or atlas streamline clusters. Using the same clustering algorithm ensures consistency and thus accurate comparability of the different clustering results.

The predetermined clustering algorithm may comprise, for a respective plurality of (tractogram and/or atlas) streamlines, assigning Q10 an initial streamline to an initial (tractogram and/or atlas) streamline cluster. The initial streamline may be chosen in any manner, for example, it may be randomly chosen from all of the streamlines (included in the tractogram or the white matter atlas). In examples, the initial streamline may be chosen according to an indexation (e.g., the indexation being ordered) of the streamlines determined when obtaining the tractogram or the white matter atlas.

The predetermined clustering algorithm may also comprise, for the respective plurality of streamlines, iteratively visiting Q20 subsequent streamlines of the respective plurality of streamlines. In other words, the predetermined clustering algorithm may traverse all other streamlines not having been chosen as the initial streamline. The visiting may be performed in any manner, for example, if the streamlines are indexed, the method may visit iteratively the indices (e.g., one-by-one) of streamlines subsequent (e.g., above or below) from the initial streamline.

For each respective subsequent streamline and with respect to a predetermined distance Q30, the predetermined clustering algorithm may also comprise computing Q310 a respective distance value between the subsequent streamline and a centroid of each already-existing streamline cluster.

By "respective subsequent streamline" it is meant a streamline visited by the predetermined clustering algorithm. By "already-existing" streamline cluster it is meant any streamline cluster created beforehand by the predetermined clustering algorithm, for example the initial streamline cluster when there is no other streamline cluster.

The centroid may be a mean streamline of a respective streamline cluster (from the tractogram or the white matter atlas) with respect to other streamlines already included (assigned, e.g., via a label) to the cluster. That is, the centroid may be a streamline (being for example a sequence of 3D points) assigned (i.e., included) in the streamline cluster which satisfies an average position with respect to other streamlines included in the cluster. The average position may be with respect to an average distance (for instance with respect to the same predetermined distance) with respect to other streamlines already included in the cluster, e.g., the centroid may be the streamline extending in the geometrical average (within some rounding errors) with respect to other streamlines already included in the cluster (for example the initial streamline in the case where the algorithm visits a subsequent streamline after the assigning Q10). The average may be a numerical average, for example, when the streamlines are indexed in the cluster. The centroid of each streamline cluster may be dynamically re-positioned when a streamline is assigned to the streamline cluster, that is, at each time when a new streamline is assigned to the streamline cluster.

The computing Q310 may be the result of computing the respective predetermined distance between the subsequent streamline and the centroid, for each already existing cluster.

Examples of the predetermined distance are now discussed.

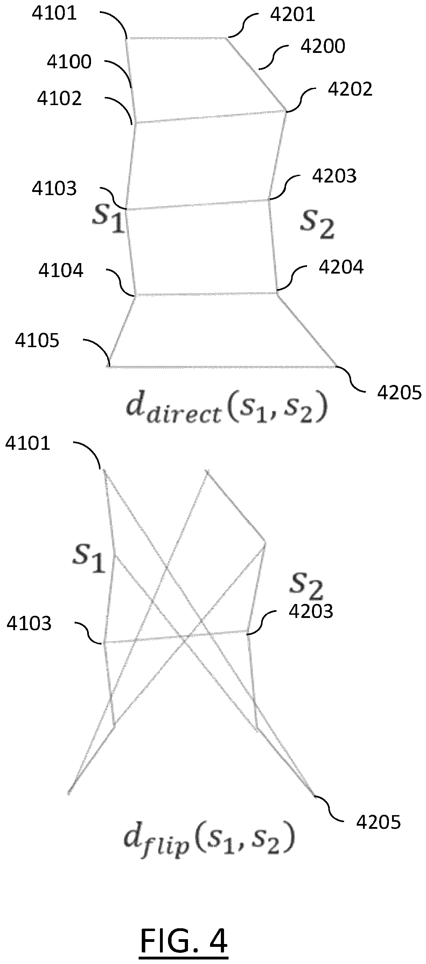

The predetermined distance may be a minimum direct-flip distance. Reference is made to FIG. 4, illustrating, on two streamlines 4100, 4200, the case where the predetermined distance may be the minimum direct-flip distance of the type:

$$MDF = \min\left(d_{direct}(f, s), d_{flip}(f, s)\right),$$

Where the predetermined distance is the minimum of the terms $d_{flip}(f, s) = d(f, s^F) = d(f^F, s)$ and $d_{direct}$ = max (dist$_{eucl}$ (s$_{1points}$, s$_{2points}$)), where $f, s$ denote two points of input streamlines s$_1$ 4100 and s$_2$ 4200.

The term $d_{direct}$ denotes the maximum of the Euclidean distance (also called direct distance) between points (s$_{1points}$, s$_{2points}$) of the two streamlines s$_1$ and s$_2$.

The points (s$_{1points}$, s$_{2points}$) may be obtained in any manner, for example by performing a same discretization of the two streamlines (uniform or not-uniform, however the same number of points for the two streamlines), or by sampling points from the two streamlines s$_1$ and s$_2$. The points may be ordered and the method may compare the distance between points having the same order. For example, the two streamlines s$_1$ and s$_2$ may have an ordered sequence of points 4101 - 4105 (for the streamline s$_1$) and 4201-4205 (for the streamline s$_2$). The direct distance $d_{direct}$ may thus compute the maximum of the Euclidean distance between two points, e.g., (4101,4201), (4102,4202) and so forth.

The term $d_{flip}(f, s)$ denotes a flip-distance term. Denoting as $f, s$ two points of each streamline, and $s^F$, $f^F$ two points corresponding to a flip in each respective streamline (or permutation in each respective streamline), the flip-distance term has the property $d_{flip}(f, s^F) = d_{flip}(f^F, s)$; in other words, the flip-distance between $f$ and $s^F$ is the same as the distance between $f^F$ and s. For example, the term $d_{flip}(f, s)$ may compute the distances between two extremum points (4101,4205) for example, the points corresponding to the first and last point respectively of each streamline s$_1$ and s$_2$, and may also compute the distances between two center points (4103,4203), that is the points corresponding to middle points of each streamline s$_1$ and s$_2$ and so forth.

The predetermined clustering algorithm may also comprise, determining Q320 a respective streamline cluster having a smallest distance value (with respect to the respective subsequent streamline) among the respective distance values computed at Q310. In the case where there are more than one streamline clusters having the smallest distance value (e.g., equidistant to the subsequent streamline up-to some tolerance), the method may chose randomly the respective streamline cluster among said streamline clusters.

If the respective (smallest) distance value is below a predetermined threshold Q330, the predetermined clustering algorithm may assign Q331 the respective subsequent streamline to the respective streamline cluster. For example, the assigning Q331 may associate the respective subsequent streamline to a data piece (e.g., such as a label) indicative of its assigning with the respective streamline cluster being determined as having the smallest distance value at Q320.

Else, the predetermined clustering algorithm may create Q332 a subsequent streamline cluster and assign the respective subsequent streamline to said subsequent streamline cluster. For example, the creating Q332 may associate the respective subsequent streamline to a data piece (e.g., such as a label) indicative of its assignation to a new cluster, i.e., part of a new set of streamlines, and thus being discriminated from other streamline clusters.

The method thus improves on the segmentation. Thanks to using the predetermined clustering algorithm, the method reduces the dimensionality of the tractogram by gathering streamlines that have a similar shape (as represented by the fulfillment of the predetermined distance) and a close location.

Moreover, when the predetermined distance is the minimum direct-flip distance, this results in that the method improves the quality of the clusters, as the predetermined clustering algorithm dissociates streamlines which are close in the middle and which separate significantly around its extrema.

Selecting the one or more first sets may comprise computing a tractogram centroid for each respective tractogram streamline cluster using a predetermined centroid computation algorithm. The tractogram centroid may be the mean tractogram streamline of a respective tractogram streamline cluster. That is, the first tractogram centroid may be a tractogram streamline assigned (i.e., included) in the tractogram streamline cluster (obtained by the predetermined clustering algorithm) which satisfies an average with respect to other tractogram streamlines included in the tractogram cluster.

Selecting the one or more first sets may also comprise computing a first atlas centroid for each respective atlas streamline cluster using the predetermined centroid computation algorithm. The first atlas centroid may be the mean atlas streamline of a respective atlas streamline cluster. That is, the first atlas centroid may be an atlas streamline assigned (i.e., included) in the atlas streamline cluster (that is, the atlas streamline cluster obtained by the same predetermined clustering algorithm used for obtaining the plurality of tractogram streamline clusters) which satisfies an average with respect to other atlas streamlines included in the atlas streamline cluster, e.g., other atlas streamlines having a label indicative of being included in the same atlas streamline cluster.

Selecting the one or more first sets may also comprise identifying each tractogram streamline cluster for which the value of the predetermined distance (e.g., the minimum direct-flip distance) between its tractogram centroid and each first atlas centroid is not above (equal or lower than) a predetermined threshold. For example, the identifying may compare the value of an absolute distance between the tractogram centroid and each first atlas centroid. The identifying may compare the value of the predetermined distance at every pair of points (resulting for example from respective discretization) between the tractogram centroid and each first atlas centroid. For example, the method may perform the identifying with the predetermined distance being the minimum direct-flip distance so as to determine if the tractogram centroid and each first atlas centroid are close in their respective middle portion and which have a separation around its endpoints not above (i.e., lower or equal than) the predetermined threshold (the threshold being for example 15mm or 20mm, the number of points of the streamline being between 20 and 50, for example 100)). The predetermined threshold may be dependent on the number of points, for example, the number of points may be higher than 100, and the predetermined threshold may be obtained by multiplying by a corresponding factor. In other words, the identified tractogram streamline clusters are thus determined as fulfilling the proximity criterion for being selected as a first set.

Selecting the one or more second sets may comprise, for each remaining tractogram streamline cluster (that is, any tractogram streamline cluster which has not been selected as a first set), determining a plurality of second atlas centroids each for a respective sectional portion of the respective plurality of sectional portions of the atlas streamline clusters. A second atlas centroid of the plurality may be the sectional portion of a mean atlas streamline (e.g., between a beginning "cross-section" and an end "cross-section") comprised in the respective sectional portion of the atlas streamline cluster.

Selecting the one or more second sets may also comprise identifying each tractogram streamline cluster for which the value of the predetermined distance between its tractogram centroid and each second atlas centroid is not above a predetermined threshold (e.g., thus equal or lower than said predetermined threshold), for the respective sectional portion. The identifying may for example compare the value of the predetermined distance between positions of the tractogram centroid and each second atlas centroid. For example, the predetermined distance may be the minimum direct-flip distance so as to determine if the tractogram centroid and each second atlas centroid are close in their respective middle portion and which have a separation around its endpoints not above the predetermined threshold (for example equal or lower than 15mm or even 20mm or more when the atlas centroid comprises for example 100 points). In other words, the identified tractogram streamline clusters are thus determined as fulfilling the proximity criterion for being selected as a second set.

This further improves the quality for segmenting anatomically accurate streamlines. Indeed, the selecting allows to determine sections of the tractogram streamline clusters (represented in the one or more second sets) which are anatomically correct, but which may have been corrupted by noise. In addition, the method may be implemented with high efficiency, as the use of centroids is computationally less expensive than having to compute distances for all of the streamlines comprised in the cluster.

Determining the plurality of second atlas centroids may comprise determining the value of a length of the tractogram centroid. The length may be the distance (e.g., Euclidean distance) between the lines connecting the two endpoints (i.e., extrema) of the tractogram centroid, in other words, the distance following the curves of the centroid.

Determining the plurality of second atlas centroids may also comprise, for each first atlas centroid having a value of the length higher than the tractogram centroid (of the remaining tractogram streamline cluster not selected as a first set), extracting one or more sectional portions of the first atlas centroid having a same value of the length as the tractogram centroid. In other words, the method may only consider two cross-sections on the first atlas centroid having the same length as the tractogram centroid as a second atlas centroid.

For example, the identifying may extract a sectional portion of the first atlas centroid from a beginning point to an intermediate point (determined by the length of the tractogram) for determining the value of the predetermined distance between the tractogram centroid and each second atlas centroid. This allows to match the length of the tractogram centroid with the length of the sectional portion of the second atlas centroid.

Extracting the one or more sectional portions of the first atlas centroid may comprise cutting iteratively the first atlas centroid using a predetermined offset (and thereby iteratively extracting different portions of the centroid moving along the length of the centroid by the predetermined offset between two consecutive iterations). In other words, the method may iteratively offset the positions at which the sectional portion of the first atlas centroid is extracted. In examples, the method may continue the iterative cutting as a function of a measure of agreement of the value of the predetermined distance between the tractogram centroid and the second atlas centroid. This allows to find the sectional portion of the second atlas centroid having a best agreement with the tractogram centroid, as the iterations may be stopped when finding the lowest distance below (or equal to) the predetermined threshold. Hence, the method may retain tractogram streamlines which may have been cut or which are incomplete.

The predetermined clustering algorithm may further comprise, after assigning all streamlines of the plurality of streamlines, re-computing the centroid of each (atlas or tractogram) streamline cluster using the predetermined centroid computation algorithm. In other words, the predetermined centroid computation algorithm may compute again a mean streamline of the streamline cluster with respect to the currently assigned streamlines. Indeed, after all the successive assignments, the centroid may have changed. Re-computing the centroid at such a time, to perform the following steps, allows a compromise between computational costs and final accuracy, as explained in the following.

The predetermined clustering algorithm may also comprise, for each streamline assigned to a respective streamline cluster, and with respect to the predetermined distance, computing a respective distance value between the streamline and the centroid of the respective streamline cluster. For example, the predetermined distance may be the minimum direct-flip distance. Additionally, the predetermined clustering algorithm may also un-assign the streamline from the respective streamline cluster if the respective distance value is above the predetermined threshold. In other words, the un-assigning removes the association of the streamline associate with the respective streamline cluster, e.g., by removing the data piece (e.g., such as a label) indicative of its assignation, or marking it with another data piece indicative of not being assigned to the respective streamline cluster.

The predetermined clustering algorithm may also comprise re-computing again the centroid of each (atlas or tractogram) streamline cluster using the predetermined centroid computation algorithm. In other words, the predetermined centroid computation algorithm may compute the mean streamline of the streamline cluster with respect to streamlines that remained assigned to the respective streamline cluster after the un-assigning.

The predetermined clustering algorithm may also comprise, for each streamline un-assigned to another respective streamline cluster, and with respect to the predetermined distance, computing a respective distance value (e.g., the minimum direct-flip distance) between the streamline and the centroid of each streamline cluster.

The predetermined clustering algorithm may also determine a streamline cluster having a smallest distance value. If the respective distance value is below the predetermined threshold (e.g., of 5mm or less), the predetermined clustering algorithm may re-assign the unassigned streamline to the determined streamline cluster. In the case where there are more than one streamline clusters having the same smallest distance value, the method may re-assign the unassigned streamline to one of said streamline clusters chosen at random. Else, the predetermined clustering algorithm may create a subsequent streamline cluster and re-assign the subsequent streamline to a subsequent streamline cluster.

This further improves the quality of the segmentation of the tractogram. Indeed, the predetermined clustering algorithm allows to reallocate streamlines which may have been misplaced, thereby resulting in a finer control on how the streamlines are clustered. Indeed, before the re-computing of the centroid of each (atlas or tractogram) streamline cluster using the predetermined centroid. The predetermined clustering algorithm including the re-computation of centroids results in a finer discretization of the streamlines where each streamline is allocated to a different cluster if it does not correspond to its original cluster.

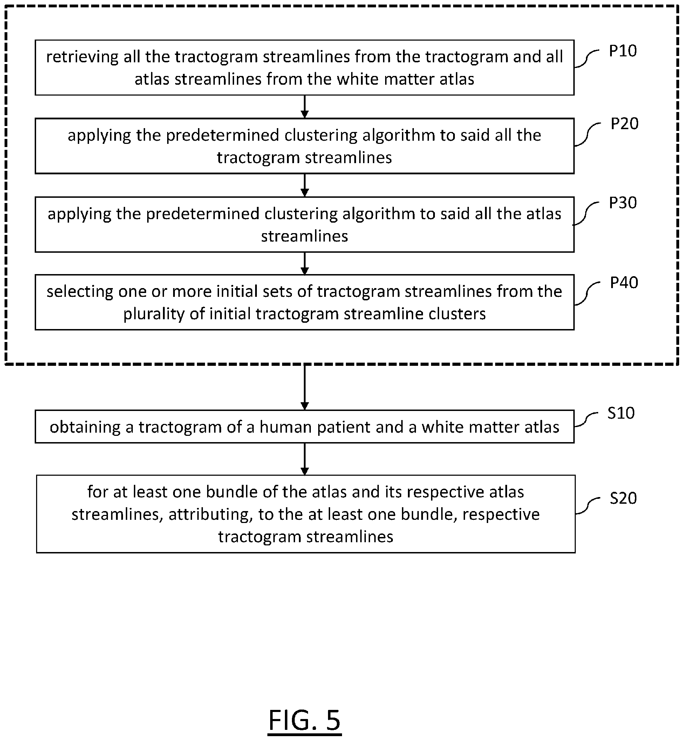

With reference to FIG. 5, the predetermined clustering algorithm may take as input a threshold value. Additionally, prior to the steps of using the predetermined clustering algorithm and to the steps of selecting the one or more first sets and the one or more second sets, the method may comprise retrieving P10 all the tractogram streamlines from the tractogram and all atlas streamlines from the white matter atlas. In other words, the method collects the set of all tractogram streamlines and all the atlas streamlines for any (e.g., all of) steps P20-P40 below.

The method may also comprise applying P20 the predetermined clustering algorithm to said (retrieved) all the tractogram streamlines to obtain an initial plurality of tractogram streamline clusters. The initial plurality of tractogram streamline clusters may be a set of initial tractogram clusters, for example, not being previously associated with any other cluster.

The method may also comprise applying P30 the predetermined clustering algorithm to said all the atlas streamlines. The predetermined clustering algorithm may obtain an initial plurality of atlas streamline clusters.

The method may also comprise selecting P40 one or more initial sets of tractogram streamlines from the plurality of initial tractogram streamline clusters. The one or more initial sets of tractogram streamlines selected in P40 may be the ones on which the attributing S20 is performed. A respective initial tractogram streamline cluster may be selected as an initial set when the respective initial tractogram streamline cluster fulfills a coarser proximity criterion with the plurality of initial atlas streamline clusters. The proximity criterion may be a criterion for determining a proximity (with respect to the shape, similarity or location) between the initial tractogram streamline clusters and a plurality of atlas streamline clusters. By "coarser proximity criterion" it is meant having a higher predetermined threshold than the proximity determined at S20. The higher predetermined threshold may be the input threshold value. For example, the predetermined threshold used at S20 may be of 5mm while the coarser proximity criterion may have a threshold of 15mm.

The using of the predetermined clustering algorithm to obtain the plurality of tractogram streamline clusters may be applied to the streamlines of at least part (e.g., all of) the one or more initial sets of tractogram streamlines. The using of the predetermined clustering algorithm may determine positions on the tractogram from which the at least part of the or more initial sets of tractogram streamlines are obtained, for example, following an initial distribution of said positions or a totally random distribution of said positions. The using of the predetermined clustering algorithm to obtain the plurality of atlas streamline clusters may still be applied to said all atlas streamlines, but also with the higher threshold value as input.

This improves the efficiency of the segmentation. Indeed, as the initial tractogram streamline clusters are selected as initial sets when the respective initial tractogram streamline cluster fulfills the coarser proximity criterion, the selected one or more initial sets are a rough (or coarse) first segmentation that removes tractogram streamlines which are located relatively far from the white matter fiber bundles (for example, outlier tractogram streamlines) or which are not located on a particular zone of interest.

The method may further comprise, to obtain the at least part of the one or more initial sets, computing a binary mask from the white matter atlas. The binary mask may comprise binary-valued voxels. The binary mask may be a grid of binary-valued voxels. A binary-valued voxel may be a volumetric element which may be identified by three dimensional coordinates (e.g., x-y-z coordinates) corresponding to the location of the binary-valued voxel in the grid. The binary-valued voxel may have, at its corresponding location, a value of one when said voxel intersects (e.g., by superposing the binary mask onto the white-matter atlas) a part of one atlas streamlines from the retrieved all white matter atlas streamlines and a value of zero otherwise.

The method may also comprise applying the binary mask to exclude each initial set of tractogram streamlines having a centroid comprising at least one point (intersected) in a voxel having a value of zero, for example a point corresponding to a point included in the streamline (e.g., in the case where the streamline is a sequence of 3D points) or a sampling of the streamline at the intersection with the voxel (in case where the streamline is a solid 3D line). In other words, the method does not perform the attributing S20 on any set of said set of tractogram streamlines.

The method may add, to the selected one or more initial sets of tractogram streamlines one or more additional (i.e., not including the already selected one or more initial sets in P40) sets of tractogram streamlines. The one or more additional sets of tractogram streamlines may be added when one or more of the retrieved (all) tractogram streamlines intersect a binary-valued voxel of the binary mask having value of one. Hence, the method also performs the attributing S20 on said set of tractogram streamlines.

This further improves the quality of the segmentation. Indeed, the use of the binary mask thus allows to re-associate tractogram streamlines which may have been cut or not taken into account when selecting P40 the one or more initial sets of tractogram streamlines.

The method may further comprise (i.e., after the attributing S20), obtaining a median number of tractogram streamlines for the one or more first sets of at least one tractogram streamlines and the one or more second sets of at least one tractogram streamline. Additionally, the method may re-attribute (that is, associating tractogram streamlines of the tractogram which are not part of the one or more first sets or the one or more second sets), in positions (or seed points) of the tractogram where the number of tractogram streamlines are below the median number of a (tractogram) streamlines, respective supplementary (tractogram or atlas) streamlines (on said positions). In examples, the method may obtain the median number of streamlines intersecting each voxel of the binary mask having a value of one. The method may perform the re-attributing on the voxel (having the value of one) with a number of seed points (indicating the positions) being equal to the median number of streamlines minus the number of streamlines intersecting the voxel. The respective supplementary (tractogram) streamlines may include one or more first supplementary (supplementary from the first sets assigned at S20) sets of at least one tractogram streamline that each correspond to a respective set of at least one atlas streamline. Additionally, the respective supplementary tractogram streamlines may further include one or more second supplementary sets of at least one tractogram streamline that each correspond to a respective sectional portion of a respective set of at least one atlas streamline. The method may re-compute the median number of tractogram streamlines and repeat the re-attribution iteratively until the median number does not change between two iterations.

This further improves the quality of the segmentation. Indeed, the method reattributes more tractogram streamlines on clusters determined to having a low number of streamlines, with respect to the median number. Moreover, the addition is computationally efficient, as it only concentrates on tractogram streamline clusters having a number of streamlines below the median number.

Examples are now discussed, with reference to FIG.s 6 to 18.

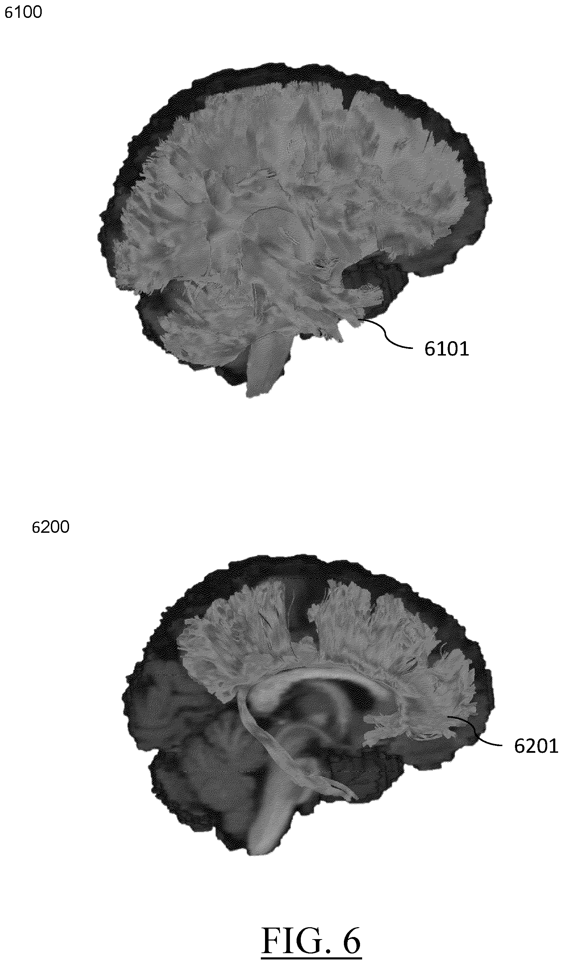

FIG. 6 shows an example of a tractogram 6100 and of a white matter atlas 6200 that can be obtained at S10. The tractogram 6100 includes tractogram streamlines 6101 (for example, millions) built using a diffusion voxel model on a diffusion MRI. The white matter atlas 5200 includes atlas streamlines which are representative of fibers of white matter. The white matter atlas 6200 has been obtained in this implementation from the Tractometer Challenge. The white matter atlas is built on multiple patients on which a tractogram has been computed and experts manually selected streamlines that belong to the anatomical bundle of interest. The white matter atlas 6200 is composed of 25 bundles of atlas streamlines 6201 acquired on a single patient, segmented using regions of interests and then being postprocessed.

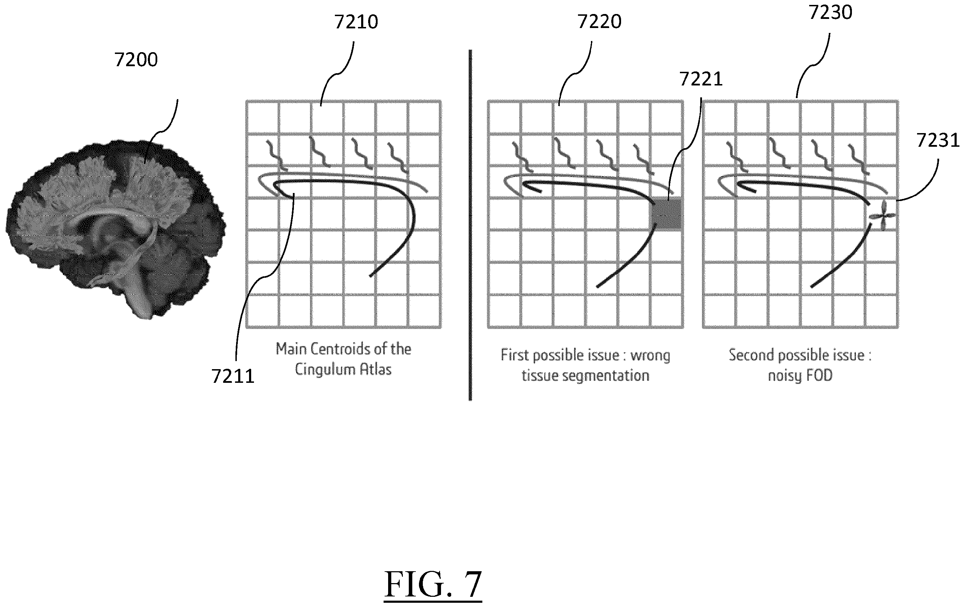

FIG. 7 illustrates some of the issues found when segmenting a tractogram 7200. For the sake of illustration, it is shown a discretization (shown as a cell grid 7210) of single tractogram streamlines, comprising a tractogram streamline 7211 belonging to a fiber of the cingulum. A first issue 7220 is a wrong labelling of the white matter / grey matter. With respect to a discretization of the tractogram (for example as a point of voxel), there is a check to ensure that this new discrete point belongs to the white matter. However, if the discrete point is labelled as grey matter (for example at cell 7221), the streamline stops thus leading to a prematurely cut streamline. A second issue 7230 is a noisy diffusion voxel model in a voxel, in fact, if the diffusion voxel model is too noisy (for example including a noisy cell 7231), the streamline cannot estimate a direction to follow and stops, once again leading to a prematurely cut streamline.

The predetermined clustering algorithm is now discussed.

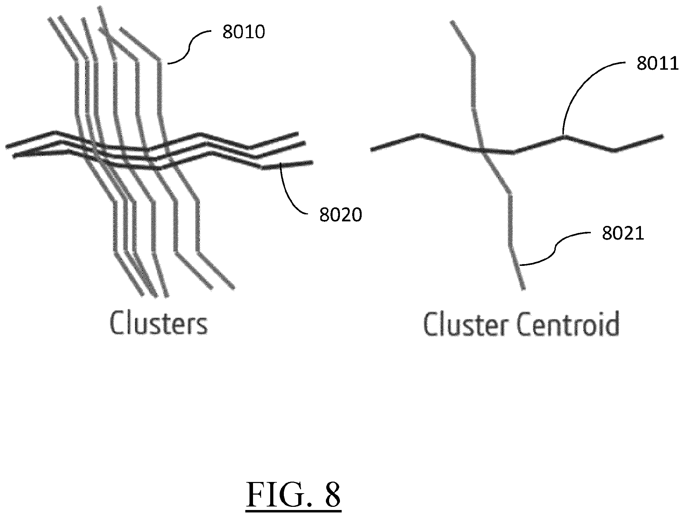

The predetermined clustering algorithm reduces the dimensionality of the tractogram by gathering streamlines that have a similar shape and a close location, into a cluster represented by a single mean streamline, also called centroid. FIG. 8 illustrates clusters 8010, 8020, and respective centroids 8011 and 8021.

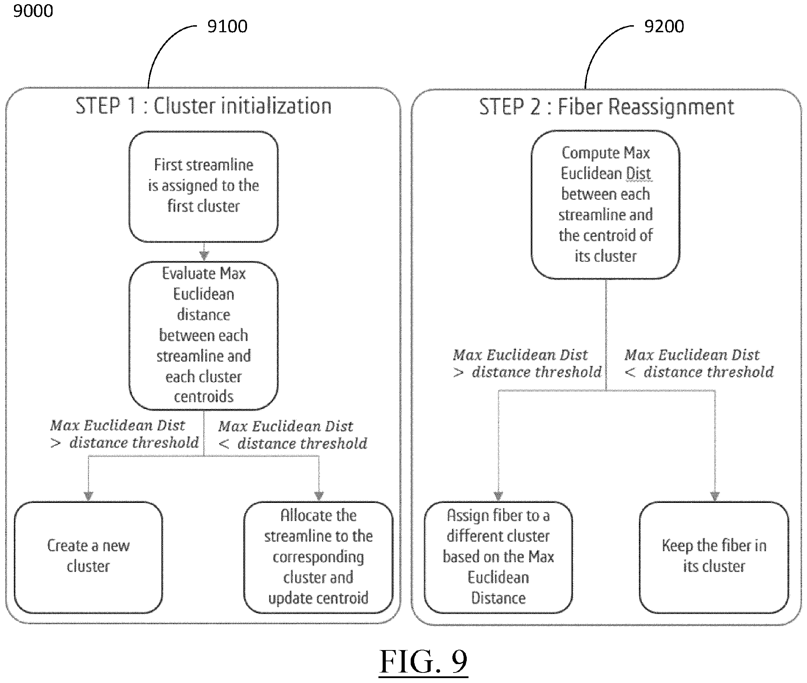

FIG. 9 illustrates a flowchart 9000 of an implementation of the predetermined clustering algorithm. The predetermined clustering algorithm may be detailed into two steps: the first step (called "Cluster initialization", which may implement steps Q10-Q30) 9100 roughly allocates (attributes) streamlines to clusters using a high level streamline discretization. The second step (also called "Tractogram streamline re-assignment") 9200 is a finer discretization of the streamlines; each streamline is reallocated to a different cluster if it does not correspond to its original cluster.

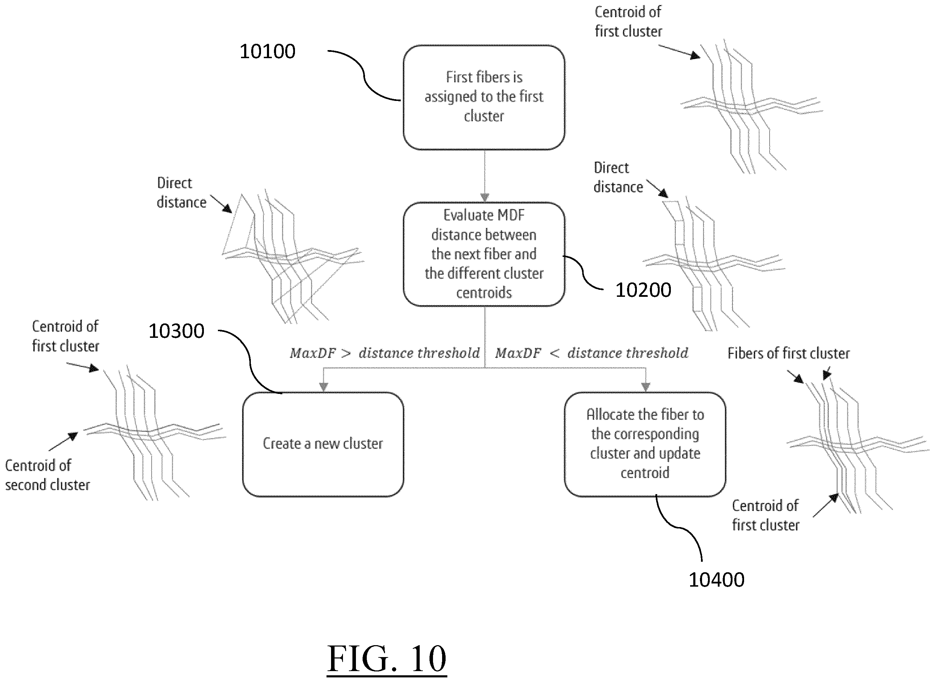

The step of cluster Initialization is now discussed, with reference to FIG. 10.

S1. Streamlines discretization.

In this implementation, all of the tractogram streamlines are discretized to a fixed number of points. The fixed number of points has been set to 10 in this implementation.

S2. Streamline assignment 10100.

The predetermined clustering algorithm randomly selects an initial streamline from the tractogram and assigns (attributes) the initial streamline to an initial cluster. The predetermined clustering algorithm also updates the centroid of the cluster.

S3. Streamline assignment 10200.

The predetermined clustering algorithm randomly selects a subsequent tractogram streamline. The predetermined clustering algorithm compares the subsequent tractogram streamline to the centroid of all the existing clusters. The metric distance used for the comparison is the minimum direct-flip distance (MDF). This metric distance is based on the computation of the Euclidean distance, between the streamline and the current centroid, point-to-point. The metric is also computed in a flip way to take into account a different point ordering. The metric is of the form

$$MDF = \min\left(d_{direct}(f,s), d_{flip}(f,s)\right).$$

When the MDF metric is computed between the streamline and all existing centroids, only the smallest MDF metric is kept to identify the closest centroid.

S4. Distance test based on a threshold.

The predetermined clustering algorithm keeps the smallest MDF metric distance by comparing it to a predetermined threshold. The predetermined threshold may be defined by a user.

If the predetermined threshold is fulfilled 10300, the predetermined clustering algorithm assigns the tractogram streamline to the cluster corresponding to the closest centroid. The predetermined clustering algorithm updates the computation of the cluster when assigning the tractogram streamline.

If the predetermined threshold is not fulfilled 10400, the predetermined clustering algorithm may assign the tractogram streamline to a new cluster.

S5. Iteration over all streamlines.

The predetermined method goes back to S3 with the next random tractogram streamline in the tractogram. The process goes on until all tractogram streamlines are assigned to a cluster.

The step of tractogram streamline re-assignment is now discussed.

In the step of cluster Initialization, the clusters have been roughly determined based on a high level discretization of the streamlines and each streamline has been allocated to a cluster. The method adds a new streamline to a cluster updates the evaluation of the centroid. In other words, the method re-computes the centroid of each streamline cluster using the predetermined centroid computation algorithm When all streamlines are assigned to different clusters, the metric distance between each streamline of the cluster and its centroid can be above the threshold defined by the user.

The predetermined clustering algorithm discretizes each streamline (e.g., selects one or more points of the sequence of 3D points representing the streamline) and each centroid are discretized to a fixed number of points, higher than the first discretization performed in the cluster initialization. The fixed number of points has been set to 100 in this implementation.

For each cluster, the MDF metric distance is computed between each of the streamline associated to the considered cluster and the centroid of the cluster. Each of the MDF metric is then compared to the previously defined threshold.

- If the distance threshold condition is fulfilled, the streamline stays in the same cluster.

- If the distance threshold condition is not fulfilled, the streamline is labelled and is removed from the cluster.

In other words, for each streamline assigned to a respective streamline cluster, and with respect to the predetermined distance, the method computes a respective distance value between the streamline and the centroid of the respective streamline cluster and un-assigns the streamline from the respective streamline cluster if the respective distance value is above the predetermined threshold.

For each cluster, the streamlines labelled are removed from the associated cluster and the centroid of each cluster is updated. In other words, the method re-computes again the centroid of each streamline cluster using the predetermined centroid computation algorithm.

For each of the streamlines that are no longer associated to a cluster (i.e., unassigned):

- the MDF metric distance is computed between the streamline and all the existing centroids.
- the smallest MDF metric is kept to identify the closest centroid
- the MDF metric is compared to the predefined threshold:

  ○ If the MDF metric is below the threshold, then the streamline is associated to the cluster associated to the identified centroid;
  ○ If the MDF metric is higher than the threshold, then a new cluster is created from the streamline.

At the end of this step, all streamlines are once again assigned to a cluster Step 5.

The process is iterative and steps 2-3-4 are computed until all streamlines are assigned to a cluster and fulfill the distance criteria based on the defined threshold. Thus, the fiber re-assignment of the predetermined clustering algorithm reallocates streamlines that would have been misplaced to another cluster, using a more finer discretization of the streamlines.

An implementation of the method, using the predetermined clustering algorithm described above is now discussed.

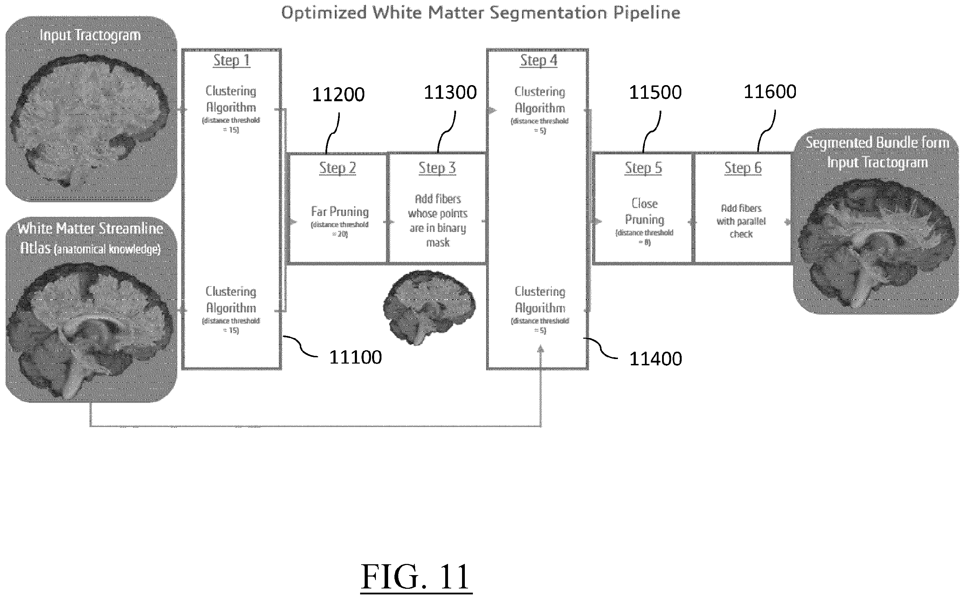

Reference is made to FIG. 11, showing a flowchart of an implementation of the method.

Far clustering of the tractogram and the atlas.

The method clusters 11100 the input tractogram and the white matter atlas (comprising, for example the cingulum) with the predetermined clustering algorithm. The method uses a distance threshold of 15mm to produce clusters with an important number of streamlines. The method may apply any kind of appropriate units.

Far pruning 11200.

The method computes the distance between each cluster centroid of the tractogram and each cluster centroid of the white matter atlas with the MDF metric distance. The method thus coarsely removes the tractogram streamlines; for example those which are of no interest, or that have nothing to do (in terms, for example, of shape or location) with the selected white matter bundle from the atlas.

For each cluster centroid of the tractogram, the method determines the smallest value of the MDF distance to identify the closest cluster centroid from the white matter bundle and also compared to a threshold, defined by the user as 20mm in our case.

If the threshold condition is fulfilled, the method keeps streamlines associated to the selected cluster centroid of the tractogram. The method thus obtains first sets corresponding to a respective set of at least one atlas streamline of the at least one bundle.

If the threshold condition is not fulfilled, the method considers tractogram as being too far from the shape and location of the white matter bundle of interest and are then discarded.

The method thus coarsely removes tractogram streamlines, for example those which are of no interest (e.g., being far in terms of shape or location) with the selected white matter bundle from the atlas.

Binary mask addition 11300

The method creates a binary mask using the white matter atlas: each voxel containing at least one atlas streamline is set to 1 otherwise 0. All streamlines associated to a cluster centroid of the tractogram which has all its points located within the binary mask and have not been retained by the method are automatically added to the remaining tractogram. The method thus includes the streamlines that could have been cut and thus forgotten by the previous steps but that could also belong to the bundle.

Close clustering 11400.

The method clusters once again remaining streamlines that have been previously selected and the white matter atlas. The new clustering uses a more constraining distance threshold, 5mm in this implementation.

Close pruning 11500

The method performs now a close pruning 11500, which is similar to the far pruning step 11200 but with a reduced distance threshold (8mm in this implementation) allowing the method to keep only the streamlines from the input tractogram that have a very close similarity of shape and location to the white matter bundle from the atlas.

Parallel study 11600

The method now adds the streamlines that would not have been kept in the previous step due to a premature cut as one or more second sets.

All centroids of the remaining tractogram that have not been kept in the close clustering step 11400 are compared in a special way to the centroids of the atlas.

For each remaining cluster centroids of the tractogram, the method performs a comparison between the considered centroid and the cluster centroid of the atlas that has been identified to be the closest one. The comparison is performed as follow:
The length of the cluster centroid of the tractogram is computed and is then reported on the cluster centroid of the atlas that has been identified to be the closest one. The identified cluster centroid of the atlas is cut from the first point to an intermediate point, in order to match the length of the cluster centroid of the tractogram.

The cut cluster centroid of the atlas is then discretized to match the fixed number of point required (100 points in this implementation). The MDF distance metric is then evaluated between the cluster centroid of the tractogram considered and the cut cluster centroid of the atlas. The MDF distance is then compared to the user defined threshold (8mm in this implementation).

If the threshold condition is fulfilled, the streamlines associated to the selected cluster centroid of the tractogram are kept. The method thus obtains second sets corresponding to a respective sectional portion of a respective set of at least one atlas streamline of the at least one bundle.

If the threshold condition is not fulfilled, the cut cluster centroid of the atlas is cut from the second point to an intermediate point so that its length match the length of the considered centroid of the cluster tractogram.

This process goes on until the threshold condition is fulfilled and the tractogram streamlines associated to the centroid of the clustered tractogram are added to the final set of streamlines as one or more second sets of tractogram streamlines or until the end of the centroid of the clustered atlas is reached and the threshold condition has not been fulfilled.

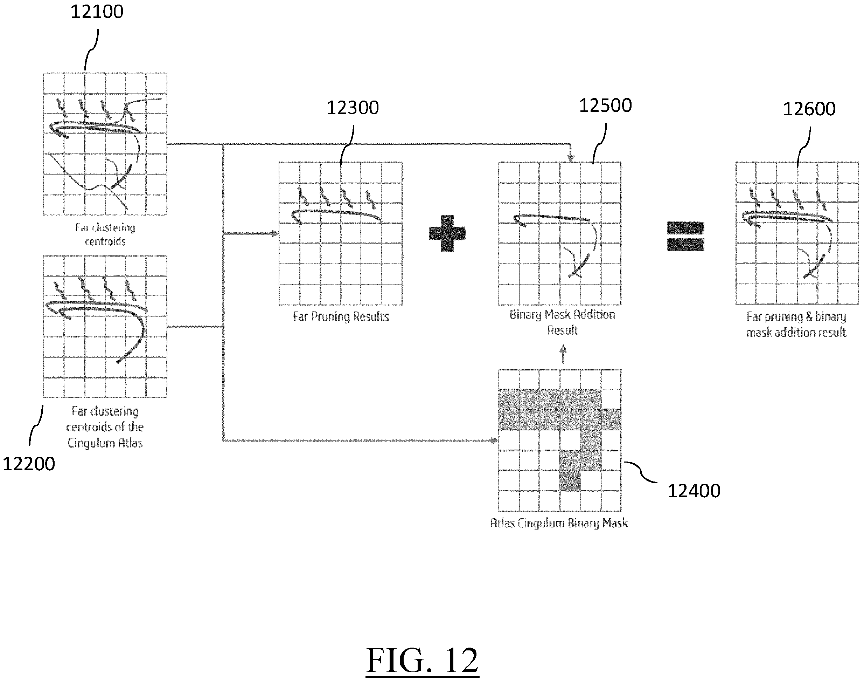

FIG. 12 illustrates the addition of the binary mask.

FIG. 12 illustrates tractogram streamlines 12000 and a corresponding streamline included in the white matter atlas 12200 (namely, representing the cingulum). After the far pruning, the retained first set of tractogram streamlines 12300 may only represent a sectional portion of the original tractogram streamline(lost due to having other tractogram streamlines which intersect the tractogram streamline). The method computes the binary mask 12400 from the white matter atlas. The binary mask 12400 shows greyed out voxels represent a value of one, when said voxel intersects a part of one atlas streamlines. The method may take this into account for adding sectional portions of the tractogram which have been lost at 12300. The method thus adds tractogram streamlines which are close to centroids of the atlas streamlines retained by the binary mask 12600.

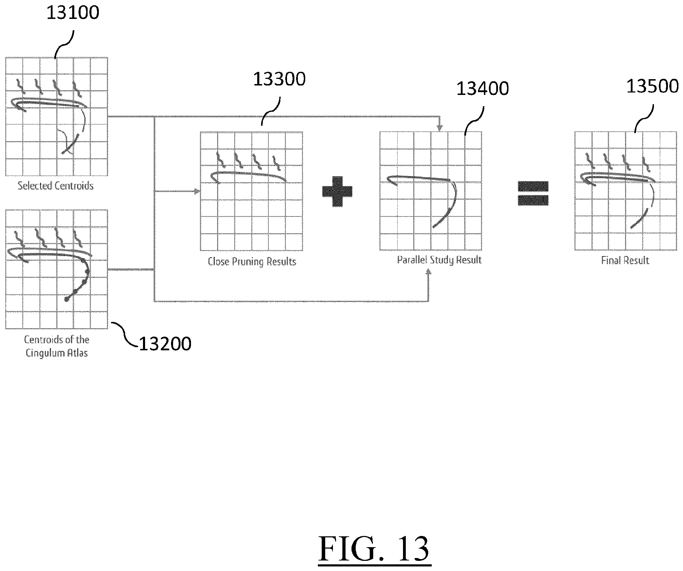

FIG. 13 illustrates an output by the method. FIG. 13 illustrates input tractogram streamlines 13100 and atlas streamlines 13200 of the cingulum. The result of the close pruning is a first set of streamlines 13300. The resulting first sets with the second sets are shown in 13400. 13500 shows all of the segmented streamlines.

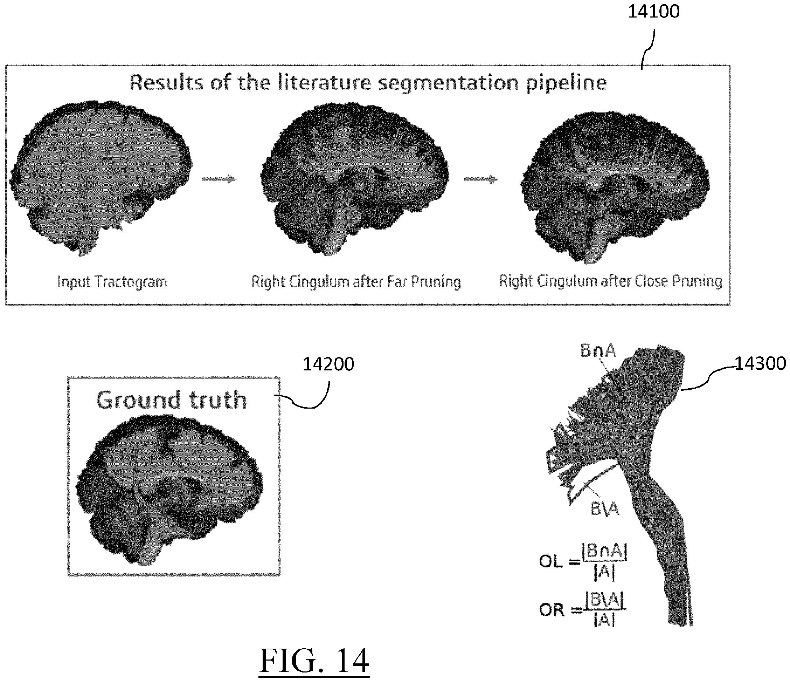

FIG. 14 illustrates the evaluation of the method.

The previously described algorithm (shown in the pipeline 14100) may be implemented and tested on the Tractometer Challenge data. The atlas extracted contains 25 bundles (that is, streams representing sets of white-matter fibers) of white matter.

The first validation is realized with a visual check. The output of the close pruning step contains a small amount of streamlines and it is difficult to find back the shape of the cingulum atlas used as input.

For more quantitative results, the method may be evaluated with some metrics in order to assess the quality of the bundle found compared to the ground truth 14200. A denotes the ground truth bundle and B a corresponding first set or second set. The overlap is the intersection of A and B. Overreach is B excluding A over the volume of A.

The metrics are the following:

- Overlap (OL) 14300: the proportion of voxels that is traversed by at least one valid streamline associated with the bundle and the ground truth bundle B over the total number of voxels within the ground truth bundle. ( Equivalent to *Recall,*

$$sensitivity = \frac{VP}{VP+FN});$$

- Overreach (OR): Fraction of voxels outside the volume of a ground truth bundle that is traversed by at least one valid streamline associated with the bundle over the total number of voxels within the ground truth bundle;

- Precision: Proportion of voxels within the volume of a ground truth bundle that is traversed by at least one valid streamline associated with the bundle over the total number of voxels associated with the bundle

$$(precision = \frac{VP}{VP+FP} \text{ or } \frac{|B \cap A|}{|B|});$$

- F1-score: How well does the bundle predicts the ground truth bundle

$$(\frac{2*recall*precision}{recall+precision}).$$

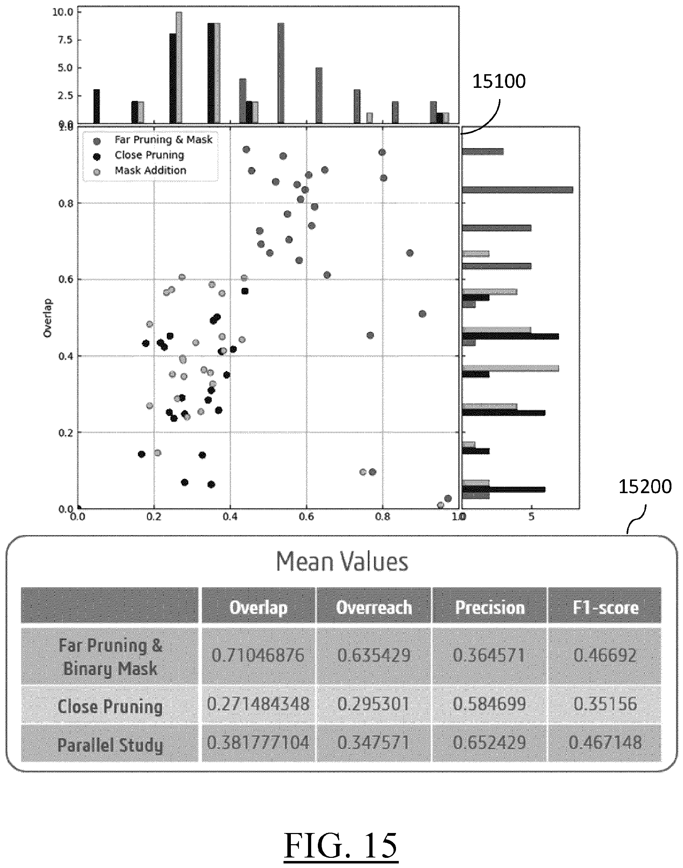

FIG. 15 illustrates the resulting metrics. The final segmentation of the proposed optimized pipeline contains much more streamlines and the ventral part of the cingulum has been segmented.

FIG. 15 shows in the graph 15100 that after the far pruning step and the binary mask addition, the segmented bundles have an average overlap and overreach quite high, 0.71 and 0.63 respectively (see also table 15200). After the close pruning step, the overlap and overreach fall to approximately 0.27 and 0.29. However, after the parallel study step, the average overlap reaches approximately 0.38 whereas the average overreach is only approximately at 0.34. This new pipeline allows to have a final segmentation where the overlap has, on average, a highest value than the overreach.

Second optimization

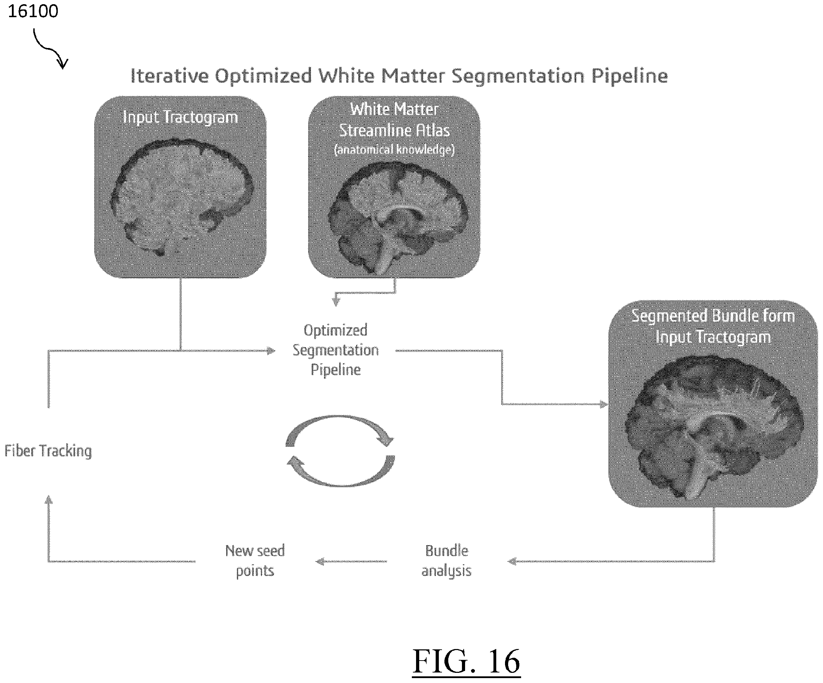

FIG. 16 shows a second optimization flowchart 16100 used for improving the number of tractogram streamlines.

The input tractogram is first segmented using a white matter atlas and the optimized white matter segmentation pipeline previously described. Once a final segmentation has been obtained, some bundle analysis is realized. The bundle analysis consists of getting the median number of streamlines per voxel within the binary mask of the final segmentation. Then new seed points are positioned within each voxels of the binary mask of the final segmentation. The number of seeds positioned within the binary is equal to the median number of streamlines previously evaluated minus the current number of streamlines within the considered voxel. If the number of streamlines going through a voxel is above the median number of streamlines evaluated, then no seed points are placed in those voxels.

The new computed streamlines are added to the final segmentation and method is run again on this new set of streamlines.

After different tests for the stopping criteria, it has been found that the process should stop when the median number of streamlines within a voxel do not change between two iterations.

This augments the number of streamlines obtained in the final segmentation to approach the ones included in the atlas. It also works for smaller bundles, as the method works with median numbers and due to the stopping criteria.

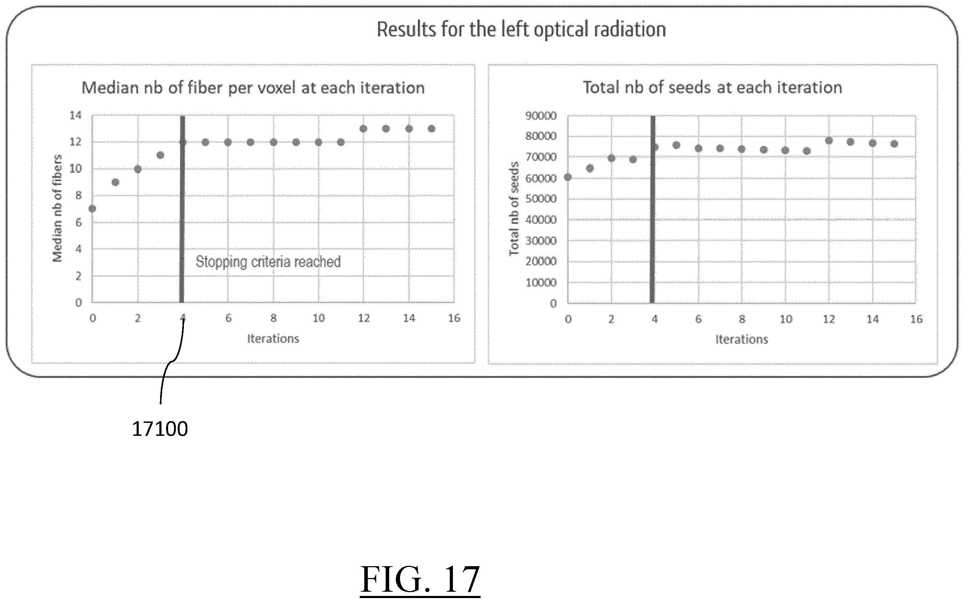

FIG. 17 shows the stopping criteria. The method may add seed points, e.g., more than 1000. In this implementation, the method may stop the addition of seed points after four iterations 17100.

Evaluation of the second optimization.

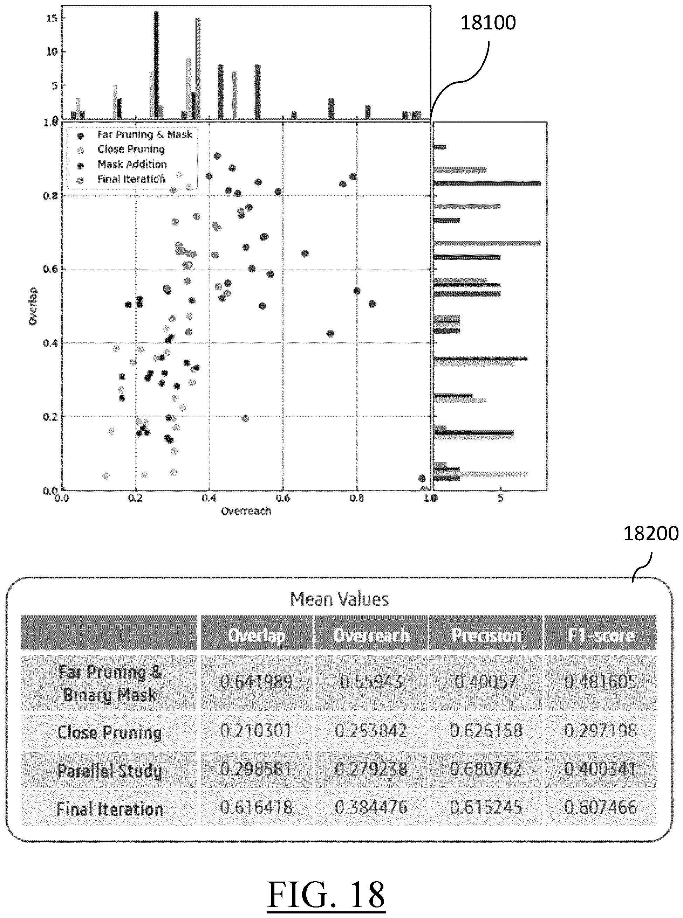

It is possible to assess the quality of the segmentation by the method using a visual validation. FIG. 18 shows a screenshot 18100, acquired on the cingulum bundle, shows that after 18 iterations, the final segmentation is much closer to the ground truth than the output of the optimized white matter segmentation algorithm.

The table 18200 shows the metrics after the final iteration for each bundle. The average overlap reaches approximately 0.61 whereas the average overreach reaches approximately 0.38. The overlap almost reaches the same level as the segmentation obtained after the initial far pruning and binary mask addition whereas the overreach is far below the value obtained after the initial far pruning and binary mask addition.

**Claims**

1. A computer-implemented method for segmenting a human patient tractogram into one or more white matter streamline bundles, comprising:

- obtaining (S10) a tractogram of a human patient, the tractogram including tractogram streamlines, and a white matter atlas including one or more bundles each including respective atlas streamlines;
- for at least one bundle of the atlas and its respective atlas streamlines, attributing (S20), to the at least one bundle, respective tractogram streamlines, the respective tractogram streamlines including one or more first sets each of at least one tractogram streamline, where each first set corresponds to a respective set of at least one atlas streamline of the at least one bundle, and the respective tractogram streamlines further including one or more second sets each of at least one tractogram streamline, where each second set corresponds to a respective sectional portion of a respective set of at least one atlas streamline of the at least one bundle.

2. The method of claim 1, wherein the attributing (S20) comprises:

   - using a predetermined clustering algorithm to obtain a plurality of tractogram streamline clusters;
   - using the predetermined clustering algorithm to obtain a plurality of atlas streamline clusters;
   - selecting the one or more first sets from the plurality of tractogram streamline clusters, a respective tractogram streamline cluster being selected as a first set when the respective tractogram streamline cluster fulfills a proximity criterion with the plurality of atlas streamline clusters; and
   - selecting the one or more second sets from the plurality of tractogram streamline clusters, a respective tractogram streamline cluster being selected as a second set when the respective tractogram streamline cluster fulfills the proximity criterion with a respective plurality of sectional portions of the atlas streamline clusters.

3. The method of claim 2, wherein:

   - selecting the one or more first sets comprises:

      ◦ computing a tractogram centroid for each respective tractogram streamline cluster using a predetermined centroid computation algorithm;
      ◦ computing a first atlas centroid for each respective atlas streamline cluster using the predetermined centroid computation algorithm; and
      ◦ identifying each tractogram streamline cluster for which the value of a predetermined distance between its tractogram centroid and each first atlas centroid is not above a predetermined threshold; and

   - selecting the one or more second sets comprises, for each remaining tractogram streamline cluster:

      ◦ determining a plurality of second atlas centroids each for a respective sectional portion of the respective plurality of sectional portions of the atlas streamline clusters; and
      ◦ identifying each tractogram streamline cluster for which the value of the predetermined distance between its tractogram centroid and each second atlas centroid is not above a predetermined threshold.

4. The method of claim 3, wherein determining the plurality of second atlas centroids comprises:

   - determining the value of a length of the tractogram centroid;
   - for each first atlas centroid having a value of the length higher than the tractogram centroid, extracting one or more sectional portions of the first atlas centroid having a same value of the length as the tractogram centroid.

5. The method of claim 3 or 4, wherein extracting the one or more sectional portions of the first atlas centroid comprises cutting iteratively the first atlas centroid using a predetermined offset.

6. The method of any one of claims 2 to 5, wherein the predetermined clustering algorithm comprises for a respective plurality of streamlines:

   - assigning (Q10) an initial streamline to an initial streamline cluster;
   - iteratively visiting (Q20) subsequent streamlines of the respective plurality of streamlines; and
   - for each respective subsequent streamline and with respect to a predetermined distance (Q30):

      ◦ computing (Q310) a respective distance value between the subsequent streamline and a centroid of each already-existing streamline cluster; and
      ◦ determining (Q320) a respective streamline cluster having a smallest distance value:

         ▪ if the respective distance value is below a predetermined threshold (Q330), assigning (Q331) the respective subsequent streamline to the respective streamline cluster;

■ else, creating (Q332) a subsequent streamline cluster and assigning the respective subsequent streamline to said subsequent streamline cluster.

7. The method of claim 6, wherein the predetermined clustering algorithm further comprises, after assigning all streamlines of the plurality of streamlines:

• re-computing the centroid of each streamline cluster using the predetermined centroid computation algorithm; and
• for each streamline assigned to a respective streamline cluster, and with respect to the predetermined distance:

○ computing a respective distance value between the streamline and the centroid of the respective streamline cluster;
○ un-assigning the streamline from the respective streamline cluster if the respective distance value is above the predetermined threshold;

• re-computing again the centroid of each streamline cluster using the predetermined centroid computation algorithm; and
• for each streamline un-assigned to another respective streamline cluster, and with respect to the predetermined distance:

○ computing a respective distance value between the streamline and the centroid of each streamline cluster;
○ determining a streamline cluster having a smallest distance value:

■ if the respective distance value is below the predetermined threshold, re-assigning the unassigned streamline to the determined streamline cluster;
■ else creating a subsequent streamline cluster and re-assigning the subsequent streamline to a subsequent streamline cluster.

8. The method of any one of claims 3 to 7, wherein the predetermined distance is a minimum direct-flip distance.

9. The method of any one of claims 2 to 8, wherein the predetermined clustering algorithm takes as input a threshold value, and prior to the steps of using the predetermined clustering algorithm and to the steps of selecting the one or more first sets and the one or more second sets, the method comprises:

• retrieving (P10) all the tractogram streamlines from the tractogram and all atlas streamlines from the white matter atlas;
• applying (P20) the predetermined clustering algorithm to said all the tractogram streamlines to obtain an initial plurality of tractogram streamline clusters;
• applying (P30) the predetermined clustering algorithm to said all the atlas streamlines; and
• selecting (P40) one or more initial sets of tractogram streamlines from the plurality of initial tractogram streamline clusters, a respective initial tractogram streamline cluster being selected as an initial set when the respective initial tractogram streamline cluster fulfills a coarser proximity criterion with the plurality of initial atlas streamline clusters;

the using of the predetermined clustering algorithm to obtain the plurality of tractogram streamline clusters being applied to the streamlines of at least part of the one or more initial sets of tractogram streamlines.

10. The method of claim 9, further comprises, to obtain the at least part of the one or more initial sets:

• computing a binary mask from the white matter atlas, the binary mask comprising binary-valued voxels, wherein a binary-valued voxel has a value of one when said voxel intersects a part of one atlas streamlines from the retrieved all white matter atlas streamlines and a value of zero otherwise; and
• applying the binary mask to exclude each initial set of tractogram streamlines having a centroid comprising at least one point in a voxel having a value of zero.

11. The method of any one of claims 1 to 10, wherein obtaining (S10) the tractogram comprises:

• obtaining a diffusion magnetic resonance image (MRI) of the brain;
• determining a diffusion voxel model from the diffusion MRI; and
• constructing the tractogram from the diffusion voxel model.

12. The method of any one of claims 1 to 11, wherein the result of the attributing is usable for diagnosing and/or treating a patient with respect to a medical condition, wherein optionally the at least one bundle comprises:

• a bundle corresponding to the cingulum fasciculus, and/or the medical condition is a condition impacting functioning of memory, such as the Alzheimer disease,

• a bundle corresponding to the corpus callosum, and/or the medical condition is the Hungtington disease,
• a bundle corresponding to optical radiations, and/or the medical condition is a condition impacting the optical nerve fibers and/or incurring an optic neuritis, such as multiple sclerosis,
• a bundle corresponding to the corticospinal tracts and/or the medical condition is the Parkinson disease, and/or
• one or more bundles usable for neurosurgical planning.

13. A computer program comprising instructions for performing the method of any of claims 1-12.

14. A computer readable storage medium having recorded thereon a computer program of claim 13.

15. A system comprising a processor coupled to a memory, the memory having recorded thereon the computer program of claim 13.

| obtaining a tractogram of a human patient and a white matter atlas | S10 |

| for at least one bundle of the atlas and its respective atlas streamlines, attributing, to the at least one bundle, respective tractogram streamlines | S20 |

## FIG. 1

## FIG. 2

assigning an initial streamline to an initial streamline cluster — Q10

iteratively visiting subsequent streamlines of the respective plurality of streamlines — Q20

for each respective subsequent streamline and with respect to a predetermined distance: — Q30

computing a respective distance value between the subsequent streamline and a centroid of each already-existing streamline cluster — Q310

determining a respective streamline cluster having a smallest distance value — Q320

distance value is below a predetermined threshold — Q330

yes

no

Q331

assigning the respective subsequent streamline to the respective streamline cluster

Q332

creating a subsequent tractogram streamline cluster and assigning the respective subsequent streamline to said subsequent streamline cluster

## FIG. 3

$$d_{direct}(s_1, s_2)$$

$$d_{flip}(s_1, s_2)$$

## FIG. 4

retrieving all the tractogram streamlines from the tractogram and all atlas streamlines from the white matter atlas — P10

applying the predetermined clustering algorithm to said all the tractogram streamlines — P20

applying the predetermined clustering algorithm to said all the atlas streamlines — P30

selecting one or more initial sets of tractogram streamlines from the plurality of initial tractogram streamline clusters — P40

obtaining a tractogram of a human patient and a white matter atlas — S10

for at least one bundle of the atlas and its respective atlas streamlines, attributing, to the at least one bundle, respective tractogram streamlines — S20

## FIG. 5

6100

6101

6200

6201

FIG. 6

EP 4 401 035 A1

7200

7210

7211

Main Centroids of the
Cingulum Atlas

7220

7221

First possible issue : wrong
tissue segmentation

7230

7231

Second possible issue :
noisy FOD

26

FIG. 7

8010

8011

8020

8021

Clusters

Cluster Centroid

FIG. 8

9000

9100

9200

**STEP 1 : Cluster initialization**

First streamline is assigned to the first cluster

Evaluate Max Euclidean distance between each streamline and each cluster centroids

*Max Euclidean Dist > distance threshold*

*Max Euclidean Dist < distance threshold*

Create a new cluster

Allocate the streamline to the corresponding cluster and update centroid

**STEP 2 : Fiber Reassignment**

Compute Max Euclidean Dist between each streamline and the centroid of its cluster

*Max Euclidean Dist > distance threshold*

*Max Euclidean Dist < distance threshold*

Assign fiber to a different cluster based on the Max Euclidean Distance

Keep the fiber in its cluster

FIG. 9

28

10100 — First fibers is assigned to the first cluster

Centroid of first cluster

Direct distance

Evaluate MDF distance between the next fiber and the different cluster centroids — 10200

Direct distance

Centroid of first cluster

$MaxDF > distance\ threshold$    $MaxDF < distance\ threshold$

Centroid of first cluster

10300 — Create a new cluster

Centroid of second cluster

Allocate the fiber to the corresponding cluster and update centroid — 10400

Fibers of first cluster

Centroid of first cluster

FIG. 10

FIG. 11

FIG. 12

13100

Selected Centroids

13300

Close Pruning Results

13400

Parallel Study Result

13500

Final Result

Centroids of the
Cingulum Atlas

13200

FIG. 13

# FIG. 14

Ground truth

14200

Results of the literature segmentation pipeline

Input Tractogram

Right Cingulum after Far Pruning

Right Cingulum after Close Pruning

14100

BnA

B\A

BnA

$$OL = \frac{|B \cap A|}{|A|}$$

$$OR = \frac{|B \setminus A|}{|A|}$$

14300

15100

15200

## Mean Values

| | Overlap | Overreach | Precision | F1-score |
|---|---|---|---|---|
| Far Pruning & Binary Mask | 0.71046876 | 0.635429 | 0.364571 | 0.46692 |
| Close Pruning | 0.271484348 | 0.295301 | 0.584699 | 0.35156 |
| Parallel Study | 0.381777104 | 0.347571 | 0.652429 | 0.467148 |

FIG. 15

34

16100

## Iterative Optimized White Matter Segmentation Pipeline

FIG. 16

FIG. 17

18100

18200

| Mean Values | | | | |
|---|---|---|---|---|
| | Overlap | Overreach | Precision | F1-score |
| Far Pruning & Binary Mask | 0.641989 | 0.55943 | 0.40057 | 0.481605 |
| Close Pruning | 0.210301 | 0.253842 | 0.626158 | 0.297198 |
| Parallel Study | 0.298581 | 0.279238 | 0.680762 | 0.400341 |
| Final Iteration | 0.616418 | 0.384476 | 0.615245 | 0.607466 |

FIG. 18

Europäisches
Patentamt
European
Patent Office
Office européen
des brevets

# EUROPEAN SEARCH REPORT

**Application Number**

EP 23 30 5037

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | GARYFALLIDIS ELEFTHERIOS ET AL: "Recognition of white matter bundles using local and global streamline-based registration and clustering", NEUROIMAGE, ELSEVIER, AMSTERDAM, NL, vol. 170, 13 July 2017 (2017-07-13), pages 283-295, XP085367689, ISSN: 1053-8119, DOI: 10.1016/J.NEUROIMAGE.2017.07.015 | 1-3,6,8, 9,11-15 | INV. G06T7/11 G06T7/136 |
| A | * page 7, right-hand column, lines 14-16 * * figure 2 * * page 285 * * abstract * * page 293, Appendix C * * page 292, right-hand column, Section 5. Conclusion * | 4,5,7,10 | |
| A | GARYFALLIDIS ELEFTHERIOS ET AL: "QuickBundles, a Method for Tractography Simplification", FRONTIERS IN NEUROSCIENCE, vol. 6, 11 December 2012 (2012-12-11), pages 1-13, XP93054233, DOI: 10.3389/fnins.2012.00175 Retrieved from the Internet: URL:https://www.ncbi.nlm.nih.gov/pmc/artic les/PMC3518823/pdf/fnins-06-00175.pdf> * page 5, left-hand column, lines 4-11 * * page 4, right-hand column, line 21 * | 1-15 | |
| | -/-- | | TECHNICAL FIELDS SEARCHED (IPC) G06T G01R |

The present search report has been drawn up for all claims

| Place of search | Date of completion of the search | Examiner |
|---|---|---|
| Munich | 15 June 2023 | Winkler, Gregor |

EPO FORM 1503 03.82 (P04C01)

Europäisches
Patentamt
European
Patent Office
Office européen
des brevets

# EUROPEAN SEARCH REPORT

Application Number

EP 23 30 5037

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | PAMELA GUEVARA ET AL: "Segmentation of Short Association Bundles in Massive Tractography Datasets Using a Multi-subject Bundle Atlas", 1 January 2011 (2011-01-01), SAT 2015 18TH INTERNATIONAL CONFERENCE, AUSTIN, TX, USA, SEPTEMBER 24-27, 2015; [LECTURE NOTES IN COMPUTER SCIENCE; LECT.NOTES COMPUTER], SPRINGER, BERLIN, HEIDELBERG, PAGE(S) 701 - 708, XP019169754, ISBN: 978-3-540-74549-5 * page 704, Section 2.3 WM Tracts Segmentation * * page 707, lines 2-3 * | 4,5 | |
| A | EP 2 102 675 B1 (KONINKL PHILIPS ELECTRONICS NV [NL]) 15 May 2013 (2013-05-15) * paragraphs [0011], [0015] * * figure 2 * | 4,5 | |
| A | CAUTERUCCIO FRANCESCO ET AL: "Improving QuickBundles to Extract Anatomically Coherent White Matter Fiber-Bundles", 1 July 2016 (2016-07-01), SAT 2015 18TH INTERNATIONAL CONFERENCE, AUSTIN, TX, USA, SEPTEMBER 24-27, 2015; [LECTURE NOTES IN COMPUTER SCIENCE; LECT.NOTES COMPUTER], SPRINGER, BERLIN, HEIDELBERG, PAGE(S) 633 - 641, XP047348130, ISBN: 978-3-540-74549-5 [retrieved on 2016-07-01] * page 635, lines 11-13 * * page 637, lines 1-8 * | 7 | TECHNICAL FIELDS SEARCHED (IPC) |

-/--

The present search report has been drawn up for all claims

| Place of search | Date of completion of the search | Examiner |
|---|---|---|
| Munich | 15 June 2023 | Winkler, Gregor |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 23 30 5037

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | ST-ONGE ETIENNE ET AL: "Fast Tractography Streamline Search", 25 September 2021 (2021-09-25), 16TH EUROPEAN CONFERENCE - COMPUTER VISION - ECCV 2020, CORNELL UNIVERSITY LIBRARY, 201 OLIN LIBRARY CORNELL UNIVERSITY ITHACA, NY 14853, PAGE(S) 82 - 95, XP047610607, [retrieved on 2021-09-25] * abstract * * figure 4 * | 10 | |
| A | RHEAULT FRANCOIS ET AL: "Bundle-specific tractography with incorporated anatomical and orientational priors", NEUROIMAGE, vol. 186, 17 November 2018 (2018-11-17), pages 382-398, XP085580460, ISSN: 1053-8119, DOI: 10.1016/J.NEUROIMAGE.2018.11.018 * abstract * | 10 | |

TECHNICAL FIELDS
SEARCHED     (IPC)

The present search report has been drawn up for all claims

| Place of search | Date of completion of the search | Examiner |
|---|---|---|
| Munich | 15 June 2023 | Winkler, Gregor |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

 

& : member of the same patent family, corresponding
    document

EPO FORM 1503 03.82 (P04C01)

**EP 4 401 035 A1**

**ANNEX TO THE EUROPEAN SEARCH REPORT
ON EUROPEAN PATENT APPLICATION NO.**

EP 23 30 5037

This annex lists the patent family members relating to the patent documents cited in the above-mentioned European search report.
The members are as contained in the European Patent Office EDP file on
The European Patent Office is in no way liable for these particulars which are merely given for the purpose of information.

15-06-2023

| Patent document cited in search report | | Publication date | Patent family member(s) | | Publication date |
|---|---|---|---|---|---|
| EP 2102675 | B1 | 15-05-2013 | CN | 101558321 A | 14-10-2009 |
| | | | EP | 2102675 A1 | 23-09-2009 |
| | | | JP | 5654237 B2 | 14-01-2015 |
| | | | JP | 2010512174 A | 22-04-2010 |
| | | | US | 2010079140 A1 | 01-04-2010 |
| | | | WO | 2008072142 A1 | 19-06-2008 |

EPO FORM P0459

For more details about this annex : see Official Journal of the European Patent Office, No. 12/82

41